# EUROPÄISCHE PATENTANMELDUNG

(11) **EP 0 771 799 A1**
(43) Veröffentlichungstag der Anmeldung: **07.05.1997**
(21) Anmeldenummer: 96117155.0
(22) Anmeldetag: 24.10.1996
(51) Int. Cl.: C07D 473/00, A61K 31/52

(54) **Purin-6-one-Derivate**

(30) Priorität: 06.11.1995 DE 19541264
(71) Anmelder: BAYER AG, 51368 Leverkusen (DE)
(72) Erfinder: Niewöhner, Ulrich, Dr., 42929 Wermelskirchen (DE); Bischoff, Erwin, Dr., 42115 Wuppertal (DE); Hütter, Joachim, Dr., 42349 Wuppertal (DE); Perzborn, Elisabeth, Dr., 42327 Wuppertal (DE); Schütz, Helmuth, Dr., 42115 Wuppertal (DE)

(57) **Zusammenfassung**

Purin-6-on-derivate werden hergestellt, indem man entsprechend substituierte Imidazol-carboxamide acyliert und anschließend zu den Purinen cyclisiert. Die Purin-6-on-derivate eignen sich als Wirkstoffe in Arzneimitteln, insbesondere zur Behandlung von cardiovaskulären Erkrankungen, Erkrankungen des Gefäßsystems sowie des Urogenitalsystems.

## Beschreibung

Die vorliegende Erfindung betrifft Purin-6-on-derivate, ein Verfahren zu ihrer Herstellung und ihre Verwendung in Arzneimitteln, insbesondere zur Behandlung von cardiovaskulären und cerebrovaskulären Erkrankungen, peripheren Gefäßerkrankungen und Krankheiten des Urogenitalsystems.

Phosphodiesterasen (PDE's) spielen eine wesentliche Rolle in der Regulation des intrazellulären cGMP und cAMP-Spiegels. Von den bisher beschriebenen Phosphodiesterase-Isoenzvmgruppen PDE I bis PDE V [Nomenklatur nach Beavo and Reifsnyder (vgl. Beavo, J.A. and Reifsnyder, D.H.: Trends in Pharmacol. Sci 11, 150 - 155 (1990))] sind die Ca-Calmodulin aktivierte PDE I, die cGMP stimulierbare PDE II und die cGMP spezifische PDE V im wesentlichen für den Metabolismus von cGMP verantwortlich. Aufgrund der unterschiedlichen Verteilung dieser cGMP metabolisierenden PDE's im Gewebe sollten selektive Inhibitoren je nach Gewebsverteilung des entsprechenden Isoenzyms die c-GMP-Spiegel im entsprechenden Gewebe anheben. Dieses kann zu einer spezifischen antiaggregatorischen, antispastischen, gefäßdilatierenden, und/oder antiarrhythmischen Wirkung führen.

Die vorliegende Erfindung betrifft jetzt Purin-6-on-derivate der allgemeinen Formel (I) 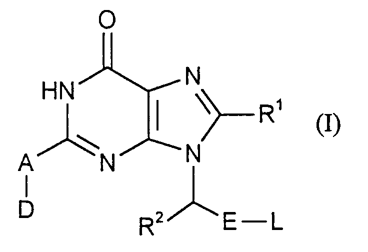 in welcher
- R¹: für Wasserstoff oder für geradkettiges oder verzweigtes Alkyl mit bis zu 8 Kohlenstoffatomen steht,
- R²: für geradkettiges oder verzweigtes Acyl mit bis zu 4 Kohlenstoffatomen steht, oder
für geradkettiges oder verzweigtes Alkyl mit bis zu 8 Kohlenstoffatomen steht, das gegebenenfalls durch Hydroxy, Azido oder durch eine Gruppe der Formel -NR³R⁴ oder -OSO₂R⁵ substituiert ist,
worin
- R³ und R⁴: gleich oder verschieden sind und
Cycloalkyl mit 3 bis 6 Kohlenstoffatomen, Wasserstoff, Formyl, oder geradkettiges oder verzweigtes Alkyl mit bis zu 6 Kohlenstoffatomen bedeuten, das gegebenenfalls durch Hydroxy, Carboxy oder durch geradkettiges oder verzweigtes Alkoxy oder Alkoxycarbonyl mit jeweils bis zu 6 Kohlenstoffatomen oder durch eine Gruppe der Formel -(CO)ₐ-NR⁶R⁷ substituiert ist,
worin
- a: eine Zahl 0 oder 1 bedeutet,
- R⁶ und R⁷: gleich oder verschieden sind und Wasserstoff, Formyl, Hydroxy, Phenyl oder geradkettiges oder verzweigtes Alkyl mit bis zu 6 Kohlenstoffatomen bedeuten, das gegebenenfalls durch Hydroxy oder durch geradkettiges oder verzweigtes Alkoxy mit bis zu 5 Kohlenstoffatomen substituiert ist,
oder
- R³ und/oder R⁴: geradkettiges oder verzeigtes Alkoxycarbonyl mit bis zu 6 Kohlenstoffatomen, Carboxy oder geradkettiges oder verzweigtes Acyl mit bis zu 6 Kohlenstoffatomen bedeuten, das gegebenenfalls durch Hydroxy oder geradkettiges oder verzweigtes Alkoxy mit bis zu 4 Kohlenstoffatomen substituiert ist
oder
- R³ und/oder R⁴: einen Rest der Formel -(CO)_{b}-T-NR⁸R⁹, -CO-R¹⁰, -SO₂R¹¹ oder -SO₂NR¹²R¹³ bedeuten, worin
- b: die oben angegebene Bedeutung von a hat und mit dieser gleich oder verschieden ist
- T: geradkettiges oder verzweigtes Alkyl mit bis zu 5 Kohlenstoffatomen bedeutet oder im Fall b ≠ 0 auch eine Bindung bedeuten kann,
- R⁸ und R⁹: die oben angegebene Bedeutung von R⁶ und R⁷ haben und mit dieser gleich oder verschieden sind
- R¹⁰: einen gesättigten, partiell ungesättigten oder ungesättigten 5- bis 7-gliedrigen Heterocyclus mit bis zu 3 Heteroatomen aus der Reihe S, N und/oder O bedeutet, der gegebenenfalls auch über die N-Funktion, durch geradkettiges oder verzweigtes Alkyl, Alkoxy oder Alkoxycarbonyl mit jeweils bis zu 4 Kohlenstoffatomen, Carboxyl, Benzyloxycarbonyl oder Hydroxy substituiert sein kann,
- R¹¹: geradkettiges oder verzweigtes Alkyl mit bis zu 5 Kohlenstoffatomen, Benzyl oder Phenyl bedeutet,
- R¹² und R¹³: gleich oder verschieden sind und Wasserstoff Phenyl oder geradkettiges oder verzweigtes Alkyl mit bis zu 6 Kohlenstoffatomen bedeuten,
oder
- R³ und R⁴: gemeinsam mit dem Stickstoffatom einen 5- oder 6-gliedrigen gesättigten, partiell ungesättigten oder ungesättigten Heterocyclus bilden, der bis zu 3 Heteroatomen aus der Reihe N, S und/oder 0 oder einen Rest -NR¹⁴ enthalten kann, und der gegebenenfalls durch Carbonyl, durch geradkettiges oder verzweigtes Alkoxycarbonyl mit bis zu 5 Kohlenstoffatomen oder durch geradkettiges oder verzweigtes Alkyl mit bis zu 5 Kohlenstoffatomen substituiert ist, das seinerseits durch Hydroxy, Carboxy oder durch geradkettiges oder verzweigtes Acyl, Alkoxy oder Alkoxycarbonyl mit jeweils bis zu 6 Kohlenstoffatomen substituiert sein kann,
worin
- R¹⁴: Wasserstoff, Carbonyl oder geradkettiges oder verzweigtes Alkyl oder Alkoxycarbonyl mit jeweils bis zu 5 Kohlenstoffatomen bedeutet,
und
- R⁵: Phenyl oder geradkettiges oder verzweigtes Alkyl mit bis zu 5 Kohlenstoffatomen bedeutet,
- A: für eine geradkettige oder verzweigte Alkylen- oder Alkenylenkette mit jeweils bis zu 6 Kohlenstoffatomen steht,
- D und L: gleich oder verschieden sind und für Aryl mit 6 bis 10 Kohlenstoffatomen oder für einen 5- bis 7-gliedrigen aromatischen, gegebenenfalls benzokondensierten Heterocyclus mit bis zu 3 Heteroatomen aus der Reihe S, N und/oder O stehen, die gegebenenfalls bis zu 3-fach gleich oder verschieden durch Halogen, Hydroxy, Nitro, Trifluormethyl, Carboxy, geradkettiges oder verzweigtes Alkyl, Alkoxy oder Alkoxycarbonyl mit jeweils bis zu 6 Kohlenstoffatomen oder durch eine Gruppe der Formel -(V)_{c}-NR¹⁵R¹⁶ und/oder -OR¹⁷ substituiert sind,
worin
- c: eine Zahl 0 oder 1 bedeutet,
- V: einen Rest der Formel -CO oder -SO₂ bedeutet,
- R¹⁵ und R¹⁶: gleich oder verschieden sind und die oben angegebene Bedeutung von R³ und R⁴ haben,
- R¹⁷: Wasserstoff, geradkettiges oder verzweigtes Alkenyl mit bis zu 8 Kohlenstoffatomen oder geradkettiges oder verzweigtes Alkyl mit bis zu 8 Kohlenstoffatomen bedeutet, das gegebenenfalls bis zu 3-fach gleich oder verschieden durch Hydroxy, Carboxyl oder geradkettiges oder verzweigtes Alkoxycarbonyl mit bis zu 5 Kohlenstoffatomen substituiert ist,
und/oder die Cyclen gegebenenfalls durch Aryl mit 6 bis 10 Kohlenstoffatomen oder durch einen 5- bis 7-gliedrigen aromatischen, gegebenenfalls benzokondensierten Heterocyclus mit bis zu 3 Heteroatomen aus der Reihe S, N und/oder O substituiert sind, die ihrerseits gegebenenfalls bis zu 2-fach gleich oder verschieden durch Halogen, Hydroxy, Nitro, Carboxyl, Trifluormethyl oder durch geradkettiges oder verzweigtes Alkyl, Alkoxy oder Alkoxycarbonyl mit jeweils bis zu 5 Kohlenstoffatomen oder durch eine Gruppe der Formel (V')_{d}-NR¹⁸R¹⁹ substituiert sind,
worin
- d: die oben angegebene Bedeutung von a hat und mit dieser gleich oder verschieden ist,
- R¹⁸ und R¹⁹: die oben angegebene Bedeutung von R³ und R⁴ haben und mit dieser gleich oder verschieden sind,
- V': die oben angegebene Bedeutung von V hat und mit dieser gleich oder verschieden ist,
und/oder
die unter D aufgeführten Ringsysteme
gegebenenfalls durch geradkettiges oder verzweigtes Acyl mit bis zu 5 Kohlenstoffatomen substituiert sind, das gegebenenfalls durch Hydroxy, geradkettiges oder verzweigtes Alkoxy mit bis zu 5 Kohlenstoffatomen oder durch eine Gruppe der Formel -NR²⁰R²¹ substituiert ist,
worin
- R²⁰ und R²¹: gleich oder verschieden und die oben angegebene Bedeutung von R³ und R⁴ haben,
oder
- D: für einen Rest der Formel 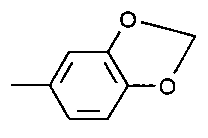 steht
und
- E: für einen Rest der Formel -CH₂-Y-Z- steht,
worin
- Y: eine Bindung oder ein Sauerstoff- oder Schwefelatom oder die -NH-Gruppe bedeutet,
- Z: eine geradkettige oder verzweigte Alkylenkette mit bis zu 5 Kohlenstoffatomen bedeutet,
und deren Tautomere und Salze.

Die erfindungsgemäßen Stoffe können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen der allgemeinen Formel (I) können in verschiedenen stereochemischen Formen auftreten, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Heterocyclus steht im Rahmen der Erfindung im allgemeinen für einen partiell ungesättigten, gesättigten oder ungesättigten 5- bis 7-gliedrigen, vorzugsweise 5- bis 6-gliedrigen Heterocyclus der bis zu 3 Heteroatome aus der Reihe S, N und/oder O enthalten kann. Beispielsweise seien genannt: Pyridyl, Pyrrolidinyl, Piperazinyl, Thienyl, Indolyl Furyl, Pyrrolyl, Thiazolyl, Oxazolyl, Imidazolyl, Morpholinyl oder Piperidyl. Bevorzugt sind Pyridyl, Thienyl, Indolyl, Furyl, Piperazinyl, Morpholinyl und Pyrrolidinyl.

Bevorzugt sind Verbindungen der allgemeinen Formel (I),

in welcher
- R¹: für Wasserstoff oder für geradkettiges oder verzweigtes Alkyl mit bis zu 6 Kohlenstoffatomen steht,
- R²: für geradkettiges oder verzweigtes Acyl mit bis zu 6 Kohlenstoffatomen steht, oder
für geradkettiges oder verzweigtes Alkyl mit bis zu 6 Kohlenstoffatomen steht, das gegebenenfalls durch Hydroxy, Azido oder durch eine Gruppe der Formel -NR³R⁴ oder O-SO₂-R⁵ substituiert ist,
worin
- R³ und R⁴: gleich oder verschieden sind und
Cyclopentyl, Cyclohexyl, Wasserstoff, Formyl oder geradkettiges oder verzweigtes Alkyl mit bis zu 5 Kohlenstoffatomen bedeuten, das gegebenenfalls durch Hydroxy, Carboxy oder durch geradkettiges oder verzweigtes Alkoxy oder Alkoxycarbonyl mit jeweils bis zu 5 Kohlenstoffatomen oder durch eine Gruppe der Formel -(CO)ₐ-NR⁶R⁷ substituiert ist,
worin
- a: eine Zähl 0 oder 1 bedeutet,
- R⁶ und R⁷: gleich oder verschieden sind und Wasserstoff Formyl, Hydroxy, Phenyl oder geradkettiges oder verzweigtes Alkyl mit bis zu 5 Kohlenstoffatomen bedeuten, das gegebenenfalls durch Hydroxy oder durch geradkettiges oder verzweigtes Alkoxy mit bis zu 4 Kohlenstoffatomen substituiert ist,
oder
- R³ und/oder R⁴: geradkettiges oder verzweigtes Alkoxycarbonyl mit bis zu 5 Kohlenstoffatomen, Carboxy oder geradkettiges oder verzweigtes Acyl mit bis zu 5 Kohlenstoffatomen bedeuten, das gegebenenfalls durch Hydroxy oder geradkettiges oder verzweigtes Alkoxy mit bis zu 3 Kohlenstoffatomen substituiert ist
oder
- R³ und/oder R⁴: einen Rest der Formel -(CO)_{b}-T-NR⁸R⁹, -CO-R¹⁰, -SO₂R¹¹ oder -SO₂NR¹²R¹³ bedeuten, worin
- b: die oben angegebene Bedeutung von a hat und mit dieser gleich oder verschieden ist
- T: geradkettiges oder verzweigtes Alkyl mit bis zu 4 Kohlenstoffatomen bedeutet oder im Fall b ≠ 0 auch eine Bindung bedeuten kann,
- R⁸ und R⁹: die oben angegebene Bedeutung von R⁶ und R⁷ haben und mit dieser gleich oder verschieden sind
- R¹⁰: Morpholinyl, Imidazolyl, Pyridyl, Piperazinyl, Piperidinyl oder Pyrrolidinyl bedeutet, die gegebenenfalls, auch über die N-Funktion, durch geradkettiges oder verzweigtes Alkyl, Alkoxy oder Alkoxycarbonyl mit jeweils bis zu 3 Kohlenstoffatomen, Carboxyl, Benzyloxycarbonyl oder Hydroxy substituiert sein können,
- R¹¹: geradkettiges oder verzweigtes Alkyl mit bis zu 4 Kohlenstoffatomen, Benzyl oder Phenyl bedeutet,
- R¹² und R¹³: gleich oder verschieden sind und Wasserstoff, Phenyl oder geradkettiges oder verzweigtes Alkyl mit bis zu 5 Kohlenstoffatomen bedeuten,
oder
- R³ und R⁴: gemeinsam mit dem Stickstoffatom einen Pyrrolidinyl-, Morpholinyl-, Imidazolyl-, Piperidinyl-, Piperazinylring bilden, die, gegebenenfalls auch über die Stickstofffunktion, durch geradkettiges oder verzweigtes Alkoxycarbonyl mit bis zu 4 Kohlenstoffatomen, Carboxyl oder durch geradkettiges oder verzweigtes Alkyl mit bis zu 4 Kohlenstoffatomen substituiert sein können, das seinerseits durch Carboxy, Hydroxy oder durch geradkettiges oder verzweigtes Acyl, Alkoxy oder Alkoxycarbonyl mit jeweils bis zu 4 Kohlenstoffatomen substituiert sein kann,
und
- R⁵: Phenyl oder geradkettiges oder verzweigtes Alkyl mit bis zu 4 Kohlenstoffatomen bedeutet,
- A: für eine geradkettige oder verzweigte Alkylen- oder Alkenylenkette mit jeweils bis zu 5 Kohlenstoffatomen steht,
- D und L: gleich oder verschieden sind und für Phenyl, Naphthyl, Pyridyl, Pyridinyl, Thienyl, Indolyl oder Furyl stehen, die gegebenenfalls bis zu 3-fach gleich oder verschieden durch Fluor, Chlor, Brom, Trifluormethyl, Nitro, Carboxy, geradkettiges oder verzweigtes Alkyl oder Alkoxycarbonyl mit jeweils bis zu 5 Kohlenstoffatomen oder durch eine Gruppe der Formel -(V)_{c}NR¹⁵R¹⁶ und/oder -OR¹⁷ substituiert sind,
worin
- c: eine Zähl 0 oder 1 bedeutet,
- V: einen Rest der Formel -CO oder -SO₂ bedeutet,
- R¹⁵ und R¹⁶: gleich oder verschieden sind und die oben angegebene Bedeutung von R³ und R⁴ haben,
- R¹⁷: Wasserstoff geradkettiges oder verzweigtes Alkenyl mit bis zu 6 Kohlenstoffatomen oder geradkettiges oder verzweigtes Alkyl mit bis zu 6 Kohlenstoffatomen bedeutet, das gegebenenfalls bis zu 2-fach gleich oder verschieden durch Hydroxy, Carboxyl oder durch geradkettiges oder verzweigtes Alkoxycarbonyl mit bis zu 4 Kohlenstoffatomen substituiert ist,
und/oder die Cyclen gegebenenfalls durch Naphthyl, Phenyl, Pyridyl, Indolyl, Thienyl, Furyl, Pyridazinyl, Pyridyl, Pyrryl oder Pyrimidyl substituiert sind, die ihrerseits gegebenenfalls durch Fluor, Chlor, Brom, Hydroxy, Nitro, Carboxyl, Trifluormethyl oder durch geradkettiges oder verzweigtes Alkyl, Alkoxy oder Alkoxycarbonyl mit jeweils bis zu 3 Kohlenstoffatomen oder durch eine Gruppe der Formel -(V')_{d}NR¹⁸R¹⁹ substituiert sind,
worin
- d: die oben angegebene Bedeutung von a hat und mit dieser gleich oder verschieden ist,
- V': die oben angegebene Bedeutung von V hat und mit dieser gleich oder verschieden ist,
- R¹⁸ und R¹⁹: die oben angegebene Bedeutung von R³ und R⁴ haben,
und/oder
die unter D aufgeführten Ringsysteme
gegebenenfalls durch geradkettiges oder verzweigtes Acyl mit bis zu 4 Kohlenstoffatomen substituiert sind, das gegebenenfalls durch Hydroxy, geradkettiges oder verzweigtes Alkoxy mit bis zu 4 Kohlenstoffatomen oder durch eine Gruppe der Formel -NR²⁰R²¹ substituiert ist,
worin
- R²⁰ und R²¹: gleich oder verschieden sind und die oben angegebene Bedeutung von R³ und R⁴ haben,
oder D für einen Rest der Formel 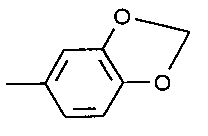 steht,
- E: für einen Rest der Formel -CH₂-Y-Z- steht,
worin
- Y: eine Bindung oder ein Sauerstoff- oder Schwefelatom oder die -NH-Gruppe bedeutet,
- Z: eine geradkettige oder verzweigte Alkylenkette mit bis zu 4 Kohlenstoffatomen bedeutet,
und deren Tautomere und Salze.

Besonders bevorzugt sind Verbindungen der allgemeinen Formel (I),
in welcher
- R¹: für Wasserstoff oder für geradkettiges oder verzweigtes Alkyl mit bis zu 4 Kohlenstoffatomen steht,
- R²: für geradkettiges oder verzweigtes Acyl mit bis zu 5 Kohlenstoffatomen steht, oder für geradkettiges oder verzweigtes Alkyl mit bis zu 5 Kohlenstoffatomen steht, das gegebenenfalls durch Hydroxy, Azido oder durch eine Gruppe der Formel-NR³R⁴ oder O-SO₂R⁵ substituiert ist,
worin
- R³ und R⁴: gleich oder verschieden sind und
Cyclopentyl, Wasserstoff, Formyl oder geradkettiges oder verzweigtes Alkyl mit bis zu 4 Kohlenstoffatomen bedeuten, das gegebenenfalls durch Hydroxy, Carboxy oder durch geradkettiges oder verzweigtes Alkoxy oder Alkoxycarbonyl mit jeweils bis zu 3 Kohlenstoffatomen oder durch eine Gruppe der Formel -(CO)ₐ-NR⁶R⁷ substituiert ist,
worin
- a: eine Zahl 0 oder 1 bedeutet,
- R⁶ und R⁷: gleich oder verschieden sind und Wasserstoff, Formyl, Hydroxy oder geradkettiges oder verzweigtes Alkyl mit bis zu 3 Kohlenstoffatomen bedeuten, das gegebenenfalls durch Hydroxy oder durch geradkettiges oder verzweigtes Alkoxy mit bis zu 3 Kohlenstoffatomen substituiert ist,
oder
- R³ und/oder R⁴: geradkettiges oder verzeigtes Alkoxycarbonyl mit bis zu 3 Kohlenstoffatomen, Carboxy oder geradkettiges oder verzweigtes Acyl mit bis zu 3 Kohlenstoffatomen bedeuten, das gegebenenfalls durch Hydroxy, Methoxy oder Ethoxy substituiert ist
oder
- R³ und/oder R⁴: einen Rest der Formel -(CO)_{b}-T-NR⁸R⁹, -CO-R¹⁰, -SO₂R¹¹ oder -SO₂NR¹²R¹³ bedeuten, worin
- b: die oben angegebene Bedeutung von a hat und mit dieser gleich oder verschieden ist
- T: geradkettiges oder verzweigtes Alkyl mit bis zu 3 Kohlenstoffatomen bedeutet
oder im Fall b ≠ 0 auch eine Bindung bedeuten kann,
- R⁸ und R⁹: die oben angegebene Bedeutung von R⁶ und R⁷ haben und mit dieser gleich oder verschieden sind
- R¹⁰: Morpholinyl, Piperidinyl, Piperazinyl oder Pyrrolidinyl bedeutet, die, gegebenenfalls auch über die N-Funktion, durch geradkettiges oder verzweigtes Alkyl, Alkoxy oder Alkoxycarbonyl mit jeweils bis zu 3 Kohlenstoffatomen, Benzyloxycarbonyl, Carboxyl oder Hydroxy substituiert sein können,
- R¹¹: geradkettiges oder verzweigtes Alkyl mit bis zu 3 Kohlenstoffatomen, Benzyl oder Phenyl bedeutet,
- R¹² und R¹³: gleich oder verschieden sind und Wasserstoff oder geradkettiges oder verzweigtes Alkyl mit bis zu 4 Kohlenstoffatomen bedeuten,
oder
- R³ und R⁴: gemeinsam mit dem Stickstoffatom einen Pyrrolidinyl-, Morpholinyl-, Imidazolyl, Piperidinyl- oder Piperazinylring bilden, die, gegebenenfalls auch über die Stickstofffunktion, durch geradkettiges oder verzweigtes Alkyl oder Alkoxycarbonyl mit jeweils bis zu 3 Kohlenstoffatomen oder Carboxyl oder durch geradkettiges oder verzweigtes Alkyl mit bis zu 3 Kohlenstoffatomen substituiert sein können, das seinerseits durch Carboxy, Hydroxy oder durch geradkettiges oder verzweigtes Acyl, Alkoxy oder Alkoxycarbonyl mit jeweils bis zu 3 Kohlenstoffatomen substituiert sein kann
und
- R⁵: Phenyl oder geradkettiges oder verzweigtes Alkyl mit bis zu 3 Kohlenstoffatomen bedeutet,
- A: für eine Ethenyl (-Vinyl) oder eine Alkylenkette mit bis zu 4 Kohlenstoffatomen steht,
worin
- D und L: gleich oder verschieden sind und für Phenyl, Naphthyl, Furyl, Thienyl, Indolyl oder Pyridyl stehen, die gegebenenfalls bis zu 2-fach gleich oder verschieden durch Fluor, Chlor, Brom, Nitro, Carboxy, Trifluormethyl, geradkettiges oder verzweigtes Alkyl oder Alkoxycarbonyl mit jeweils bis zu 4 Kohlenstoffatomen oder durch eine Gruppe der Formel -(V)_{c}NR¹⁵R¹⁶ und/oder -OR¹⁷ substituiert sind,
worin
- c: eine Zahl 0 oder 1 bedeutet,
- V: einen Rest der Formel -CO oder -SO₂ bedeutet,
- R¹⁵ und R¹⁶: gleich oder verschieden sind und die oben angegebene Bedeutung von R³ und R⁴ haben,
- R¹⁷: Wasserstoff, geradkettiges oder verzweigtes Alkenyl mit bis zu 4 Kohlenstoffatomen oder geradkettiges oder verzweigtes Alkyl mit bis zu 4 Kohlenstoffatomen bedeutet, das gegebenenfalls bis zu 2-fach gleich oder verschieden durch Hydroxyl, Carboxyl oder durch geradkettiges oder verzweigtes Alkoxycarbonyl mit bis zu 3 Kohlenstoffatomen substituiert ist
und/oder die Cyclen gegebenenfalls durch Phenyl, Pyrimidyl, Pyridazinyl, Pyridyl, Thienyl oder Furyl substituiert sind, die ihrerseits gegebenenfalls durch Fluor, Chlor, Brom, Hydroxy, Nitro, Carboxyl, Trifluormethyl oder durch geradkettiges oder verzweigtes Alkyl, Alkoxy oder Alkoxycarbonyl mit jeweils bis zu 3 Kohlenstoffatomen oder durch eine Gruppe der Formel -(V')_{d}NR¹⁸R¹⁹ substituiert sind,
worin
- d: die oben angegebene Bedeutung von a hat und mit dieser gleich oder verschieden ist,
- V': die oben angegebene Bedeutung von V hat und mit dieser gleich oder verschieden ist,
- R¹⁸ und R¹⁹: die oben angegebene Bedeutung von R³ und R⁴ haben,
und/oder
- D: gegebenenfalls durch geradkettiges oder verzweigtes Acyl mit bis zu 3 Kohlenstoffatomen substituiert ist, das gegebenenfalls durch Hydroxy, geradkettiges oder verzweigtes Alkoxy mit bis zu 3 Kohlenstoffatomen oder durch eine Gruppe der Formel -NR²⁰R²¹ substituiert ist,
worin
- R²⁰ und R²¹: gleich oder verschieden sind und die oben angegebene Bedeutung von R³ und R⁴ haben,
- D: für einen Rest der Formel 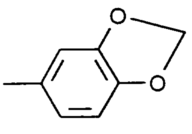 steht,
- E: für einen Rest der Formel -CH₂-Y-Z- steht,
worin
- Y: eine Bindung oder ein Sauerstoff- oder Schwefelatom oder die -NH-Gruppe bedeutet,
- Z: eine Alkylenkette mit bis zu 4 Kohlenstoffatomen bedeutet
und deren Tautomere und Salze.

Ganz besonders bevorzugt sind Verbindungen der allgemeinen Formel (I),
in welcher
- L: für Phenyl steht, das gegebenenfalls durch Fluor, Chlor, Methyl oder Methoxy substituiert ist, R¹ für Wasserstoff, Methyl oder Ethyl steht und R² für CH₃, Isopropyl oder für die Gruppen 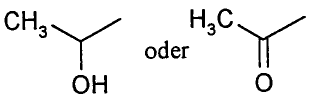 steht.

Außerdem wurden Verfahren zur Herstellung der erfindungsgemäßen Verbindungen der allgemeinen Formel (I) gefunden, dadurch gekennzeichnet, daß man
[A] zunächst Verbindungen der allgemeinen Formel (II) 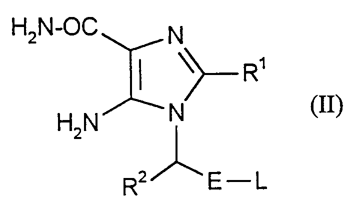 in welcher
   R¹, R², E und L die angegebene Bedeutung haben,
   durch Umsetzung mit Verbindungen der allgemeinen Formel (III)

   D-A-CO-Cl (III)

   in welcher
   A und D die angegebene Bedeutung haben,
   in inerten Lösemitteln und in Anwesenheit einer Base,
   in die Verbindungen der allgemeinen Formel (IV) 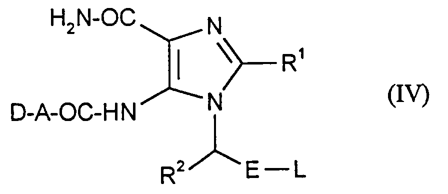 in welcher
   A, D, E, L, R¹ und R² die angegebene Bedeutung haben,
   überführt,
   und in einem zweiten Schritt in inerten Lösemitteln und in Anwesenheit einer Base cyclisiert,
   oder
[B] Verbindungen der allgemeinen Formel (II) mit Verbindungen der allgemeinen Formel (V)

   D-A-CO₂R²² (V)

   in welcher
   D und A die oben angegebene Bedeutung haben und
   R²² für C₁-C₄-Alkyl steht
   in Alkoholen und in Anwesenheit einer Base direkt umsetzt,
   und gegebenenfalls die unter R² aufgeführten Substituenten durch Folgereaktionen wie Acylierung, Oxidation, Substitution und/oder Reduktion einführt bzw. derivatisiert,
   und ebenso die oben unter D und L aufgeführten Substituenten nach üblichen Methoden einführt und/oder variiert.

Das erfindungsgemäße Verfahren kann durch folgendes Formelschema beispielhaft erläutert werden:

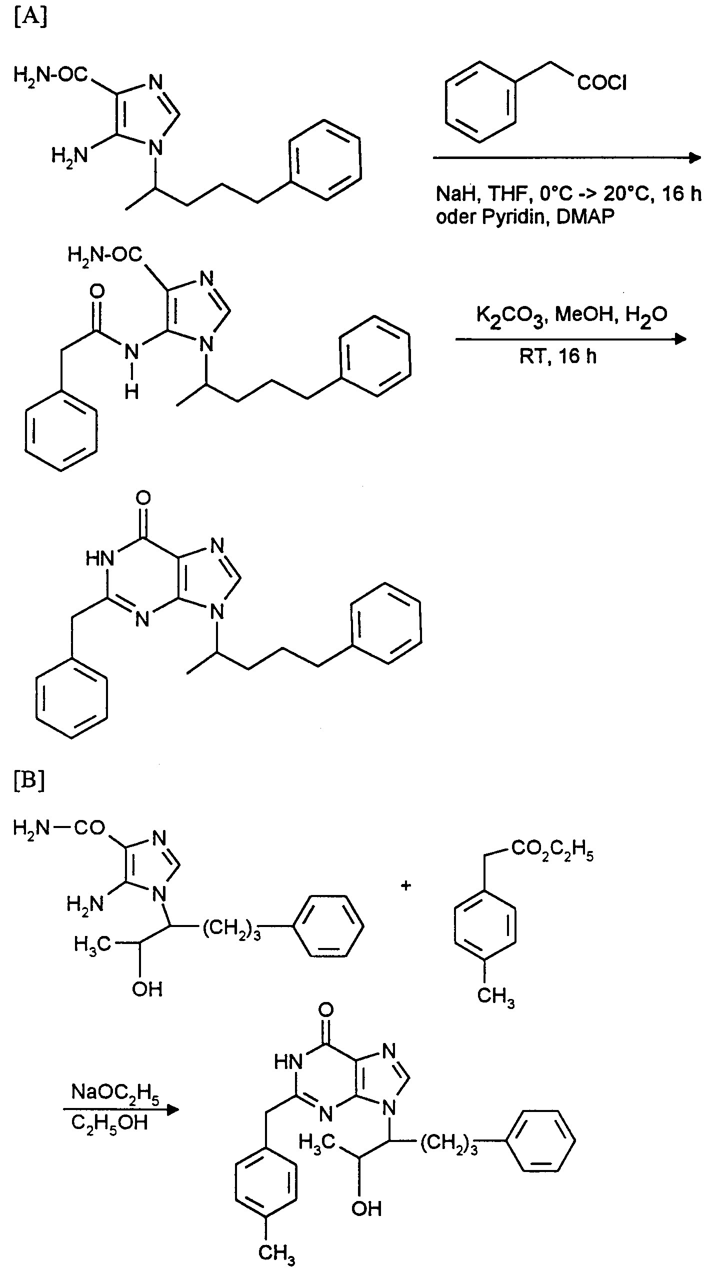

Für den ersten Schritt des Verfahrens [A] eignen sich inerte organische Lösemittel, die sich unter den Reaktionsbedingungen nicht verändern. Hierzu gehören bevorzugt Ether wie beispielsweise Diethylether, Dioxan, Tetrahydrofuran oder Glykoldimethylether oder Toluol, Hexamethylphosphorsäuretriamid und Pyridin. Selbstverständlich ist es möglich, Gemische der Lösemittel einzusetzen. Besonders bevorzugt sind Tetrahydrofuran, Toluol und Pyridin.

Als Basen eignen sich im allgemeine Alkalihydride oder -alkoholate, wie beispielsweise Natriumhydrid oder Kalium-tert.butylat, oder cyclische Amine, wie beispielsweise Piperidin, Pyridin, Dimethylaminopyridin oder C₁-C₄-Alkylamine, wie beispielsweise Triethylamin. Bevorzugt sind Natriumhydrid, Pyridin und/oder Dimethylaminopyridin.

Die Base wird im allgemeinen in einer Menge von 1 mol bis 4 mol, bevorzugt von 1,2 mol bis 3 mol jeweils bezogen auf 1 mol der Verbindungen der allgemeinen Formel (II) eingesetzt.

Die Reaktionstemperatur kann im allgemeinen in einem größeren Bereich variiert werden. Im allgemeinen arbeitet man in einem Bereich von -20°C bis 200°C, bevorzugt von 0°C bis 100°C.

In einer Variante wird die Umsetzung in Pyridin, dem eine katalytische Menge DMAP zugesetzt wird, durchgeführt. Gegebenenfalls kann noch Toluol zugefügt werden.

Als Lösemittel für die Cyclisierung eignen sich die üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Als Basen eignen sich für die Cyclisierung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt sind Kaliumcarbonat und Natriumhydroxid.

Bei der Durchführung der Cyclisierung wird die Base im allgemeinen in einer Menge von 2 bis 6 mol, bevorzugt von 3 bis 5 mol bezogen auf 1 mol der Verbindungen der Formel (IV), eingesetzt.

Die Cyclisierung wird im allgemeinen in einem Temperaturbereich von 0°C bis 160°C, bevorzugt bei der Siedetemperatur des jeweiligen Lösemittels durchgeführt.

Die Cyclisierung wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Lösemittel für das Verfahren [B] eignen sich die oben aufgeführten Alkohole, wobei Ethanol bevorzugt ist.

Als Basen für das Verfahren [B] eignen sich Alkoholate, wie beispielsweise Natriummethanolat, -ethanolat, -isopropylat oder Kalium-tert.-butylat. Bevorzugt ist Natriumethanolat.

Die Base wird in einer Menge von 2 mol bis 8 mol, bevorzugt von 3 mol bis 6 mol jeweils bezogen auf 1 mol der Verbindungen der allgemeinen Formel (II) eingesetzt.

Die Ketone werden ausgehend von den entsprechenden Hydroxyverbindungen nach bekannten Methoden (Swern-Oxidation oder Colling-Oxidation) hergestellt.

Die Variationen der Substituenten an den Aromaten werden nach bekannten Methoden durchgeführt.

Die enantiomerenreinen Verbindungen sind nach üblichen Methoden, beispielsweise durch Chromatographie der racemischen Verbindungen der allgemeinen Formel (I) auf chiralen Phasen oder durch die Verwendung chiraler Ausgangsverbindungen zugänglich.

Die Verbindungen der allgemeinen Formel (II) sind neu und können beispielsweise hergestellt werden, indem man
2-Amino-2-cyanoacetamid der Formel (VI) 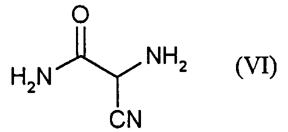 mit Verbindungen der allgemeinen Formel (VII)

R¹C(OC₂H₅)₃ (VII)

in welcher
R¹ die oben angegebene Bedeutung hat,
umsetzt und in einem zweiten Schritt mit Verbindungen der allgemeinen Formel (VIII) 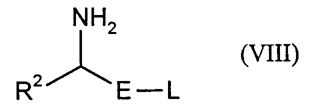 in welcher
R², E und L die angegebene Bedeutung haben,
in inerten Lösemitteln umsetzt.

Als Lösemittel für die einzelnen Schritte der Verfahren eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethylen, Trichlorethylen oder Chlorbenzol, oder Essigester, Dimethylformamid, Hexamethylphosphorsäuretriamid, Acetonitril, Aceton oder Dimethoxyethan. Ebenso ist es möglich, Gemische der genannten Lösemittel zu verwenden. Besonders bevorzugt ist Acetonitril.

Das erfindungsgemäße Verfahren wird im allgemeinen in einem Temperaturbereich von 0°C bis +180°C, bevorzugt von +30°C bis +150°C durchgeführt.

Die erfindungsgemäßen Verfahrenschritte werden im allgemeinen bei Normaldruck durchgeführt. Es ist aber auch möglich, bei Überdruck oder bei Unterdruck zu arbeiten (z.B. in einem Bereich von 0,5 bis 5 bar).

Die Verbindung der Formel (VI) ist bekannt [vgl. F.I. Logemann, G. Shaw, Chemistry and Industry, 1980 (13), 541-542].

Die Verbindungen der allgemeinen Formeln (III) und (VII) sind an sich bekannt oder nach üblichen Methoden herstellbar.

Die Amine der allgemeinen Formel (VIII) sind bekannt oder können nach bekannten Methoden hergestellt werden [vgl. L.R Krepski et al., Synthesis, 1986, 301-303].

Die Verbindungen der allgemeinen Formel (IV) sind neu und können wie oben beschrieben hergestellt werden.

Die erfindungsgemäßen Verbindungen der allgemeinen Formel (I) zeigen ein nicht vorhersehbares, wertvolles pharmakologisches Wirkspektrum.

Sie inhibieren entweder eine oder mehrere der c-GMP metabolisierenden Phosphodiesterasen (PDE I, PDE II und PDE V). Dies führt zu einem differenzierten Anstieg von c-GMP. Eine Erhöhung des c-GMP-Spiegels, kann zu einer antithrombotischen, vasodilatorischen und/oder antiarrhythmischen Wirkung führen. Die differenzierende Wirkung wird von der Verteilung der Isoenzyme im Gewebe mitbestimmt.

Außerdem verstärken die erfindungsgemäßen Verbindungen die Wirkung von Substanzen, wie beispielsweise EDRF (Endothelium derived relaxing factor) und ANP (atrial natriuretic peptide), die den cGMP-Spiegel steigern.

Sie können daher in Arzneimitteln zur Behandlung von cardiovaskulären Erkrankungen wie beispielsweise zur Behandlung des Bluthochdrucks, neuronaler Hypertonie, stabiler und instabiler Angina, peripheren und kardialen Gefäßerkrankungen, von Arrhythmien, zur Behandlung von thromboembolischen Erkrankungen und Ischämien wie Myokardinfarkt, Hirnschlag, transistorischen und ischämischen Attacken, Angina pectoris, periphere Durchblutungsstörungen, Verhinderung von Restenosen nach Thrombolysetherapie, percutaner transluminaler Angioplastie (PTA), percutan transluminalen Koronarangioplastien (PTCA) und Bypass eingesetzt werden. Die relaxierende Wirkung auf glatter Muskulatur macht sie geeignet für die Behandlung von Erkrankungen des Urogenitalsystems wie Prostatahypertrophie, Impotenz und Inkontinenz. Weiterhin können sie auch Bedeutung für cerebrovaskuläre Erkrankungen haben.

### Aktivität der Phosphordiesterasen (PDE's)

Die c-GMP stimulierbare PDE II, die c-GMP hemmbare PDE III und die cAMP spezifische PDE IV wurden entweder aus Schweine- oder Rinderherzmyokard isoliert. Die Ca²⁺-Calmodulin stimulierbare PDE I wurde aus Schweineaorta, Schweinehirn oder bevorzugt aus Rinderaorto isoliert. Die c-GMP spezifische PDE V wurde aus Schweinedünndarm, Schweineaorta, humanen Blutplättchen und bevorzugt aus Rinderaorta gewonnen. Die Reinigung erfolgte durch Anionenaustauschchromatographie an MonoQ^{R} Pharmacia im wesentlichen nach der Methode von M. Hoey and Miles D. Houslay, Biochemical Pharmacology, Vol. 40, 193-202 (1990) und C. Lugman et al. Biochemical Pharmacology Vol. 35 1743-1751 (1986).

Die Bestimmung der Enzymaktivität erfolgt in einem Testansatz von 100 µl in 20 mM Tris/HCl-Puffer pH 7,5 der 5 mM MgCl₂, 0,1 mg/ml Rinderserumalbumin und entweder 800 Bq ³HcAMP oder ³HcGMP enthält. Die Endkonzentration der entsprechenden Nucleotide ist 10⁻⁶ mol/l. Die Reaktion wird durch Zugabe des Enzyms gestartet, die Enzymmenge ist so bemessen, daß während der Inkubationszeit von 30 min ca 50% des Substrates umgesetzt werden. Um die cGMP stimulierbare PDE II zu testen, wird als Substrat ³HcAMP verwendet und dem Ansatz 10⁻⁶ mol/l nicht markiertes cGMP zugesetzt. Um die Ca-Calmodulinabhängige PDE I zu testen, werden dem Reaktionsansatz noch CaCl₂ 1 µM und Calmodulin 0,1 µM zugesetzt. Die Reaktion wird durch Zugabe von 100 µl Acetonitril, das 1 mM cAMP und 1 mM AMP enthält, gestoppt. 100 µl des Reaktionsansatzes werden auf der HPLC getrennt und die Spaltprodukte "Online" mit einem Durchflußscintillationszähler quantitativ bestimmt. Es wird die Substanzkonzentration gemessen, bei der die Reaktionsgeschwindigkeit um 50% vermindert ist. Zusätzlich wurde zur Testung der "Phosphodiesterase [³H] cAMP-SPA enzyme assay" und der "Phosphodiesterase [³H] cGMP-SPA enzyme assay" der Firma Amersham Life Science verwendet. Der Test wurde nach dem vom Hersteller angegebenen Versuchsprotokoll durchgeführt. Für die Aktivitätsbestimmung der PDE2 wurde der [³H] cAMP SPA assay verwendet, wobei dem Reaktionsansatz 10⁻⁶ M cGMP zur Aktievierung des Enzyms zugegeben wurde. Für die Messung der PDE1 wurden Calmodulin 10⁻⁷ M und CaCl₂ 1µM zum Reaktionsansatz zugegeben. Die PDE5 wurde mit dem [³H] cGMP SPA assay gemessen.

| Inhibition der Phosphodiesterasen in vitro | | | |
|---|---|---|---|
| Bsp.-Nr. | PDE I IC₅₀ [nM] | PDE II IC₅₀ [nM] | PDE V IC₅₀ [nM] |
| 1 | 500 | 200 | 300 |
| 33 | 3000 | 100 | 800 |
| 34 | ∼500 | 20 | 500 |
| 69 | 3000 | 60 | 500 |
| 80 | 100 | 80 | 1000 |
| 82 | 100 | 20 | 500 |
| 83 | 300 | 20 | 500 |
| 84 | 100 | 20 | 1000 |
| 100 | 1000 | 4 | 1000 |
| 121 | 500 | 3 | 1000 |
| 122 | 500 | 4 | 1000 |
| 138 | 500 | 1 | 500 |

Die Verbindungen wurden auf antihypertensive Aktivität am narkotisierten Schwein untersucht.

Die erektionsauslösende Wirkung wurde am narkotisierten Kaninchen gemessen. (C.G. Stief et al. World Journal Urology 1990, S. 233-236).

Die Substanzen wurden in Dosierungen 0,03 bis 10 mg/kg direkt in den Corpus cavernosum, intraduodenal, rektal, oral, transdermal oder intravenös appliziert.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös.

Im allgemeinen hat es sich als vorteilhaft erwiesen, bei intravenöser Applikation Mengen von etwa 0,01 bis 10 mg/kg, vorzugsweise etwa 0,1 bis 10 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Ausgangsverbindungen

1.) Die Amine (Formel VII / R² = Alkyl) werden aus den entsprechenden substituierten Phenethylhalogeniden durch Addition an den entsprechenden β-Ketoester, Verseifung und Decarboxylierung und reduktiver Aminierung der entsprechenden Ketone mit Natrium-cyanoborhydrid [vgl. Lane, Synthesis, 1975, 135-146] hergestellt.
2.) Die Hydroxyamine (Formel VII / R² = Hydroxy subst. Alkyl) werden in einer Eintopfreaktion aus einem Aldehyd oder Keton durch Addition von Trimethylsilylcyanid, Umsetzung der entsprechenden silylierten Cyanhydrine mit Grignardreagenzien und Reduktion der Imine mit Natriumborhydrid hergestellt [vgl. Krepski, Jensen, Heilmann, Rasmussen, Synthesis, 1986, 301-103].
3.) 1-substituierte 5-Amino-imidazol-carboxamide (Formel (II)) 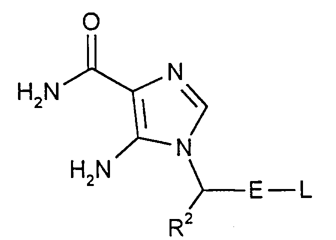 Synthese aus 2-Amino-2-cyano-acetamid (I.I. Logemann und G. Shaw, Chem. Ind. (London) 541, 1980) und dem entsprechenden Amin (siehe 1.)) bzw. Hydroxyamin (siehe 2.)) nach P.R. Birkett, C.B. Chapler und G. Mackenzie, Synthesis 1991, 157-159 oder G. Shaw, R.W. Warrener, N.D. Butler und R.K. Ralph, J. Chem. Soc. 1959, 1644-1655.

### Herstellungsbeispiele

### Darstellung der Purin-6-one

### Methode A:

22 mmol 1-substituiertes-5-amino-imidazol-4-carboxamid werden mit 1,76 g (44 mmol) Natriumhydrid (60%ig) in 300 ml abs. THF 30 Minuten refluxiert. Nach dem Abkühlen auf 0°C werden 40 mmol des entsprechenden Säurechlorids in 50 ml abs. THF zugetropft. Es wird über Nacht bei Raumtemperatur gerührt und nach zutropfen von 20 ml Methanol einrotiert. Der Rückstand wird durch Flashchromatographie über Kieselgel (Eluens: CH₂Cl₂/MeOH-Gemisch) gereinigt und man erhält die 5-Acylamino-imidazol-4-carboxamide, die direkt weiter umgesetzt werden.

10 mmol des 5-Acylamino-imidazol-4-carboxamids und 5,52 g (40 mmol) K₂CO₃ werden in 100 ml Methanol und 30 ml Wasser über Nacht refluxiert. Durch Zugabe von 2n Salzsäure wird auf pH 1 bis 2 angesäuert. Das Methanol wird im Vakuum abgezogen, es werden 100 ml Wasser zugegeben und 2 mal mit je 100 ml Essigsäureethylester ausgeschüttelt. Nach Trocknen der vereinigten organischen Phasen über Na₂SO₄ wird einrotiert und der Rückstand durch Flashchromatographie über Kieselgel (Eluens: CH₂Cl₂/MeOH-Gemisch) gereinigt. Die das Produkt enthaltenden Fraktionen werden vereinigt und eingedampft. Feste Rückstände werden aus Essigsäureethylester oder Essigsäureethylester/Diethylether umkristallisert.

### Methode B:

Zu einer Lösung von 3 mmol des 1-substituierten-5-amino-imidazol-4-carboxamids und 50 mg DMAP in 20 ml abs. Pyridin werden bei Raumtemperatur 3,3 mmol des entsprechenden Säurechlorids in wenig abs. THF getropft. Nach 30 Minuten bei Raumtemperatur wird noch ca. 5 Stunden bei 50°C gerührt (DC-Kontrolle). Der Ansatz wird eingedampft, der Rückstand wird in 20 ml CH₂Cl₂ aufgenommen und mit 20 ml 2 n HCl gewaschen. Nach Trocknen der organischen Phase über Na₂SO₄ wird eingedampft und der Rückstand durch Flashchromatographie über Kieselgel (Eluens: CH₂Cl₂/MeOH-Gemisch) gereinigt. Man erhält die 5-Acylamino-imidazol-4-carboxamide, die direkt weiter wie bei Methode A beschrieben, umgesetzt werden.

### Methode C:

2 mmol 1-substituiertes-5-amino-imidazol-4-carboxamids und 8 mmol des entsprechenden Esters werden in 20 ml einer Natriumethanolat-Ethanol-Lösung (hergestellt aus 0,23 g (10 mmol) Natrium und 20 ml absolutem Ethanol) 18 Stunden refluxiert. Nach dem Abkühlen wird mit Eisessig neutralisiert und eingedampft. Der Rückstand wird durch Flashchromatographie gereinigt und die das Produkt enthaltenden Fraktionen werden vereinigt und eingedampft. Feste Rückstände werden aus Essigsäureethylester oder Essigsäureethylester/Ether umkristallisiert.

In den Tabellen 1 und 2 sind die nach Methode A, B oder C hergestellten Purin-6-one zusammengestellt.

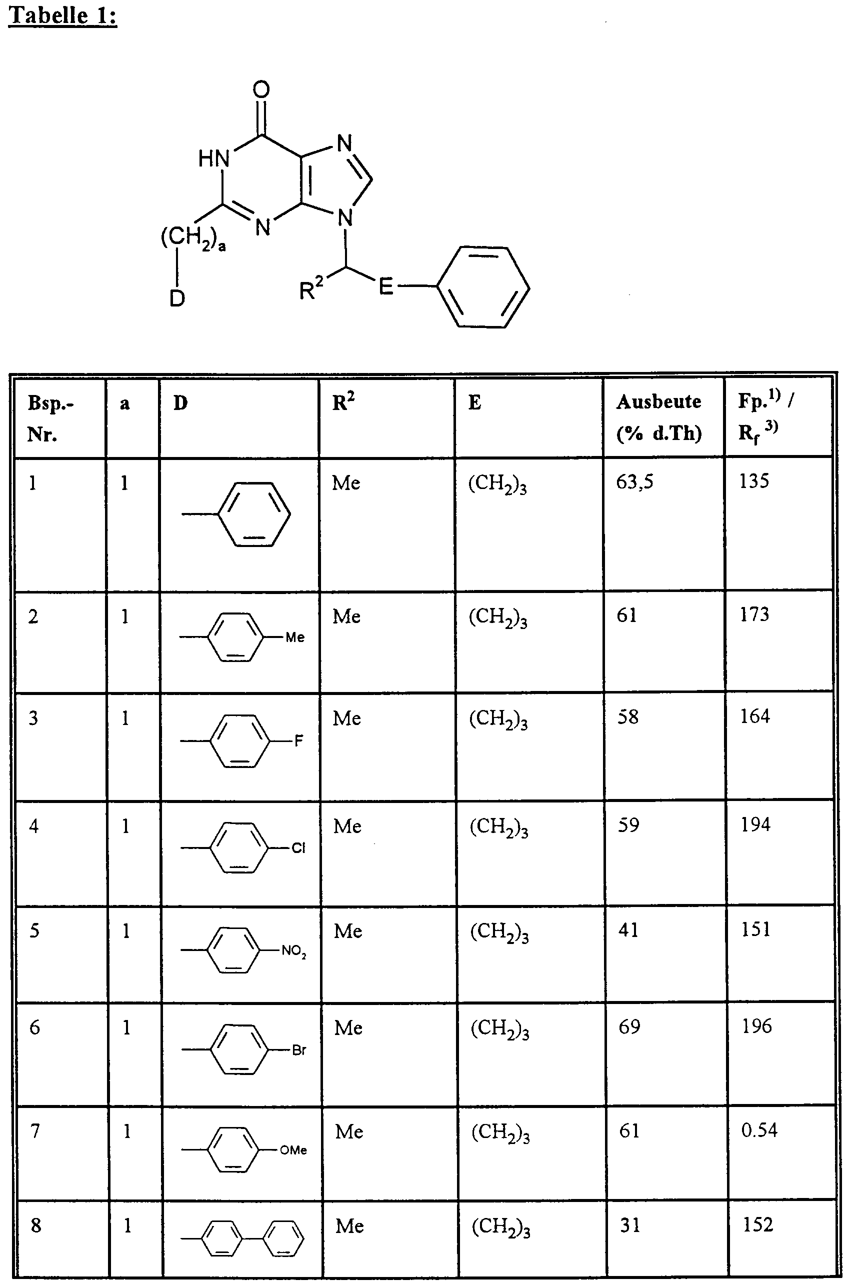

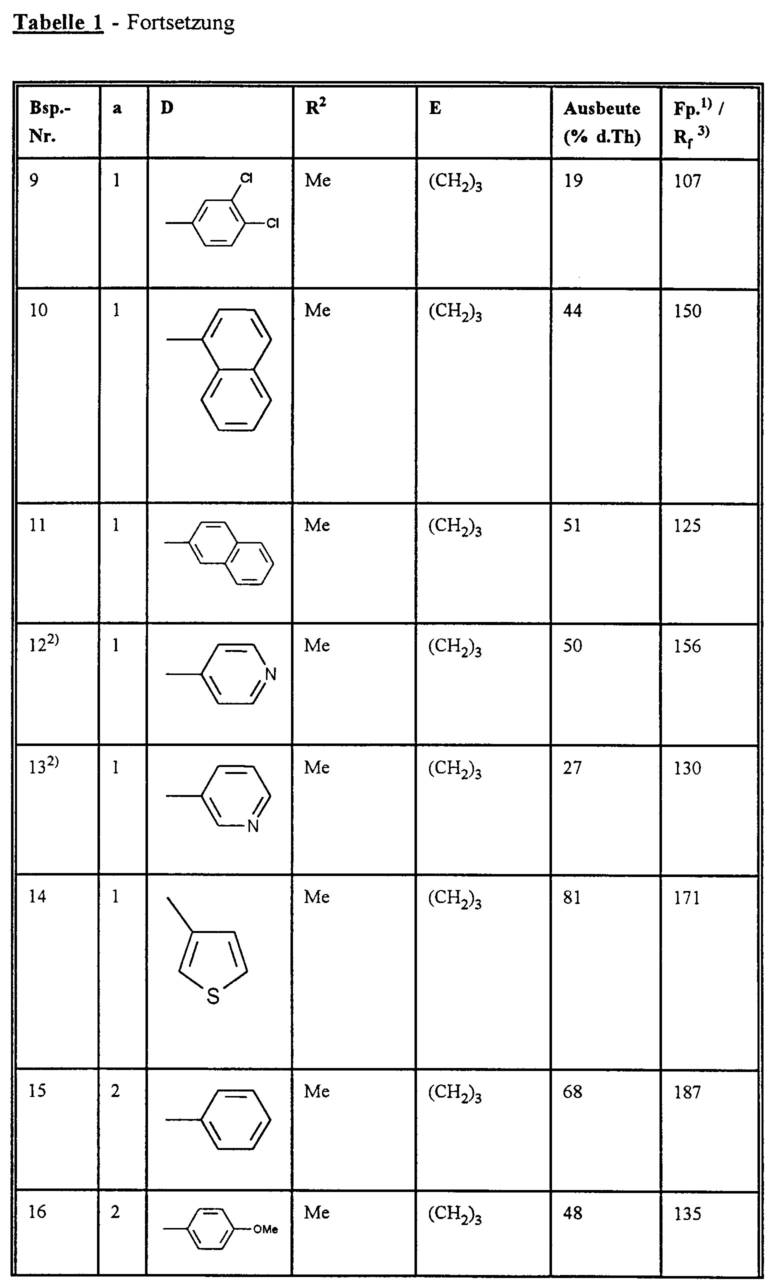

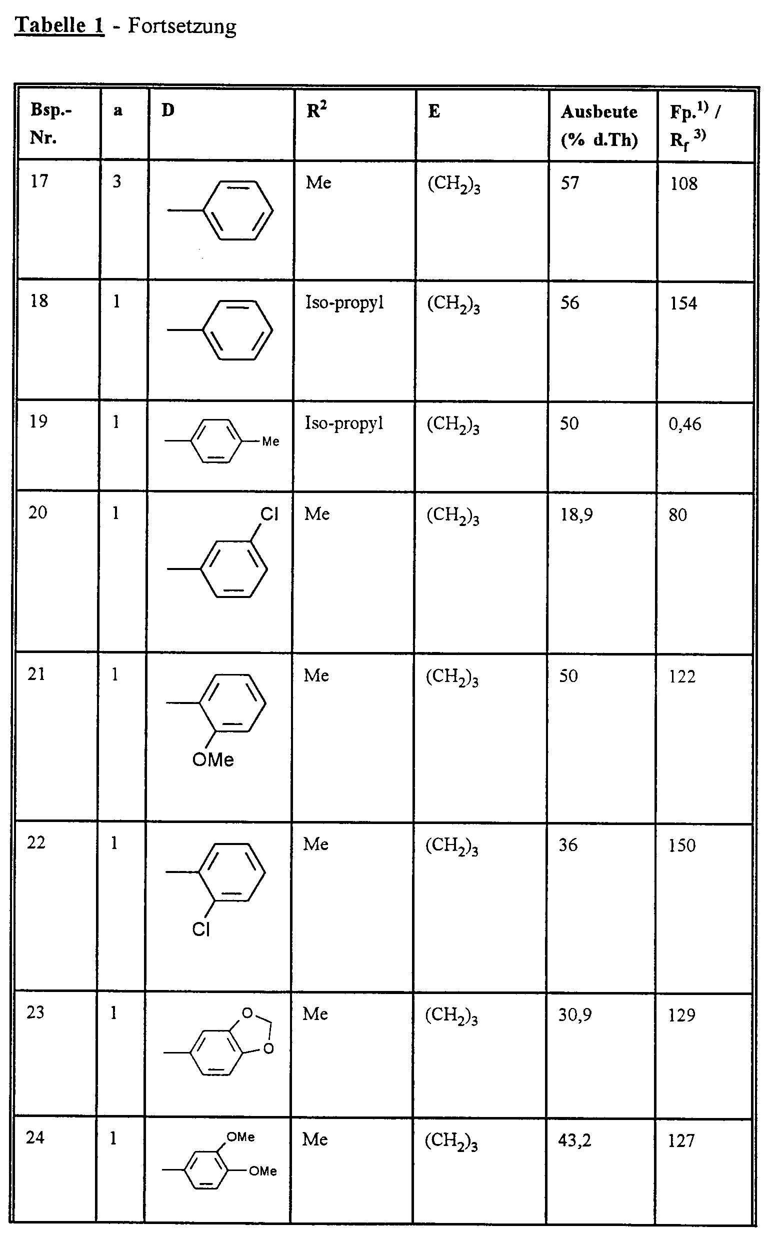

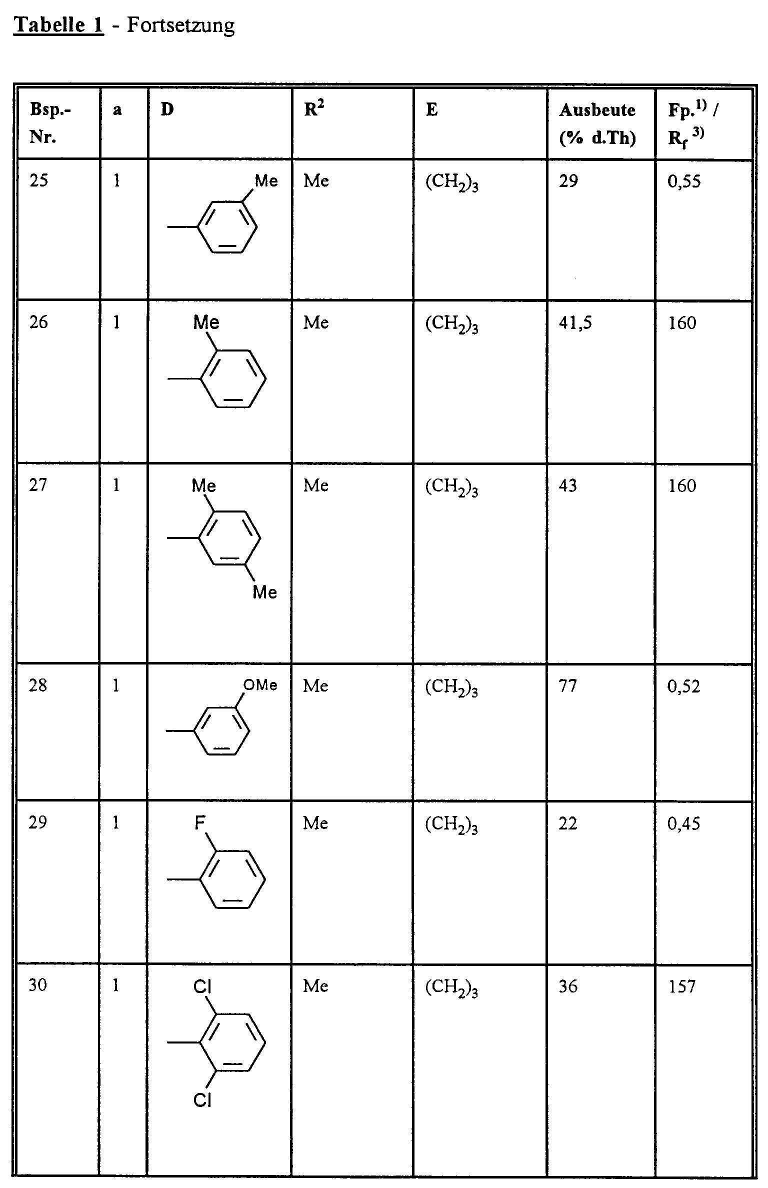

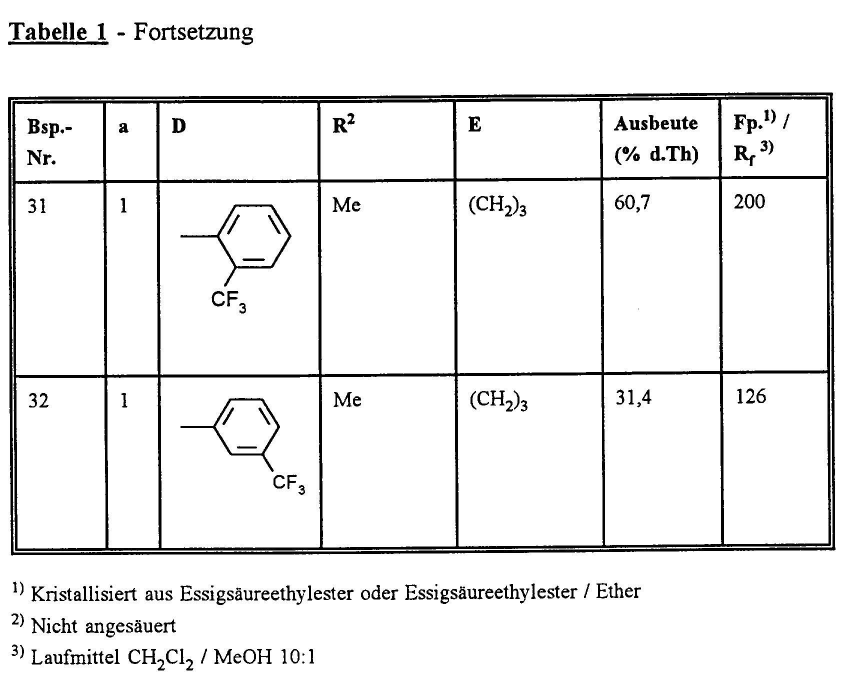

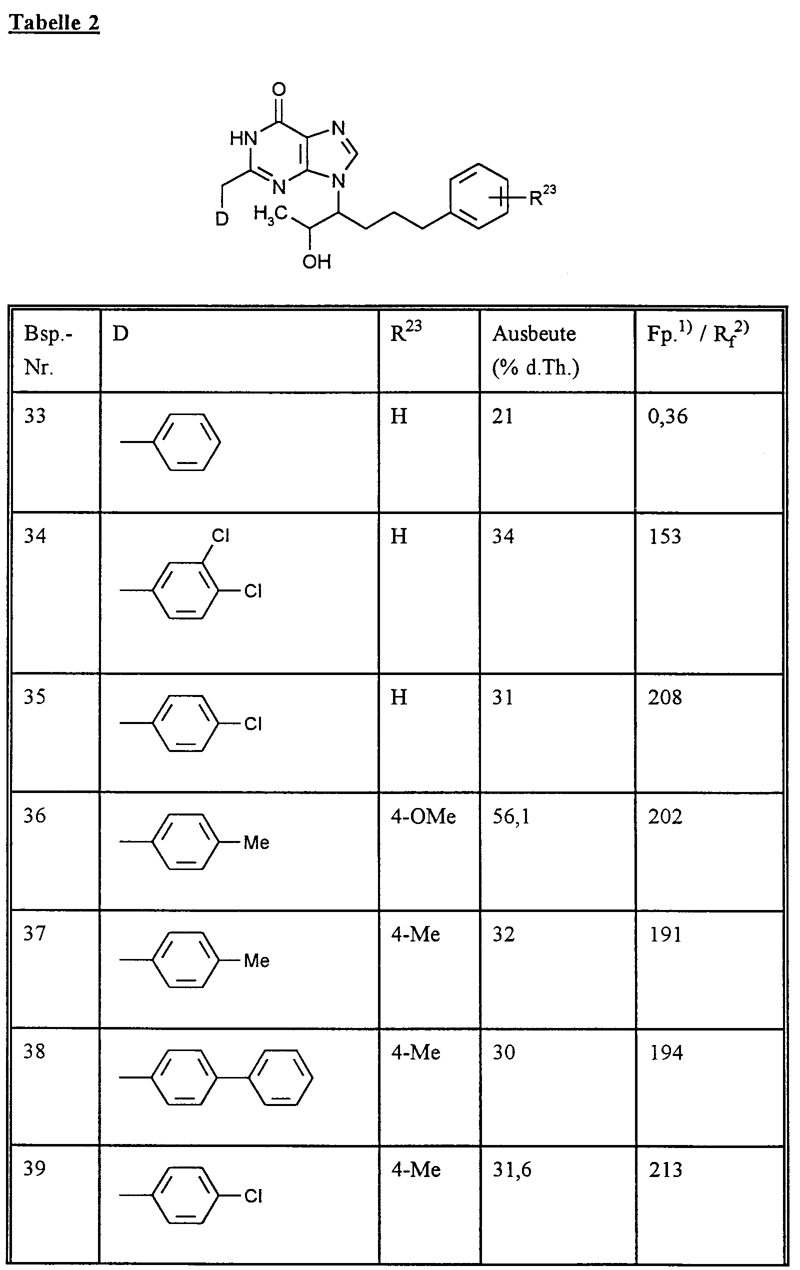

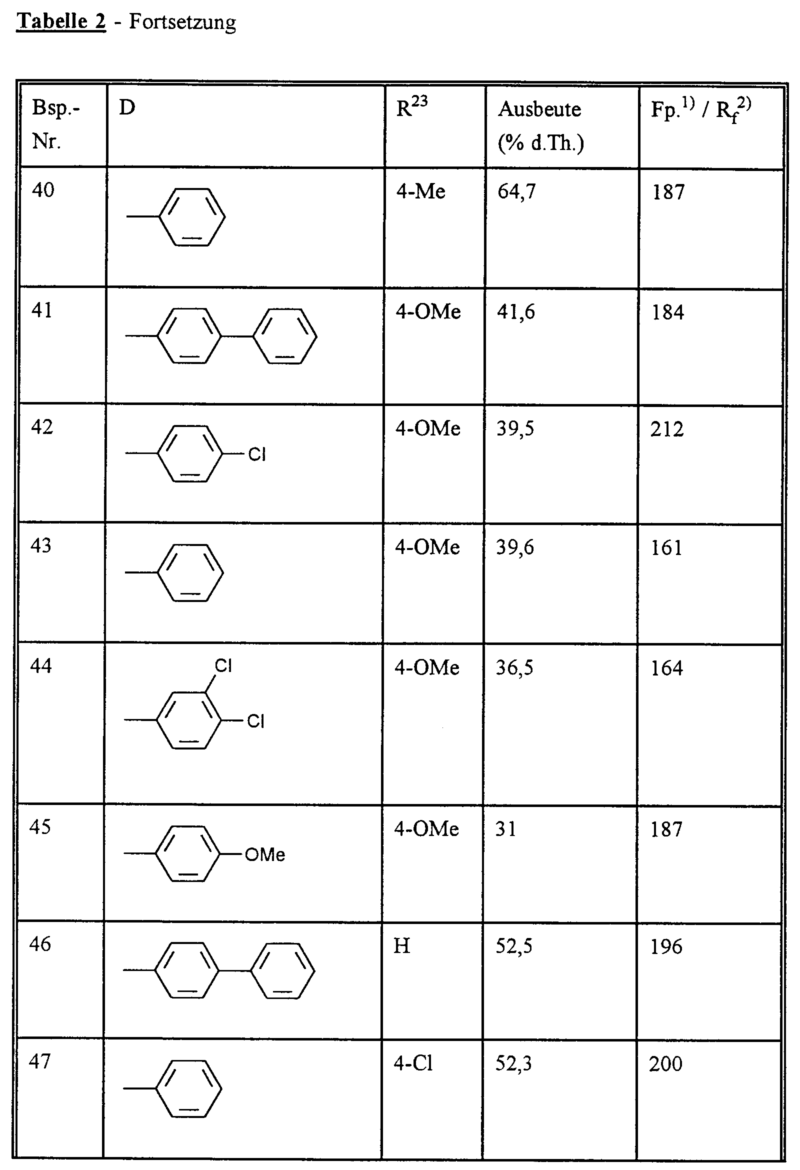

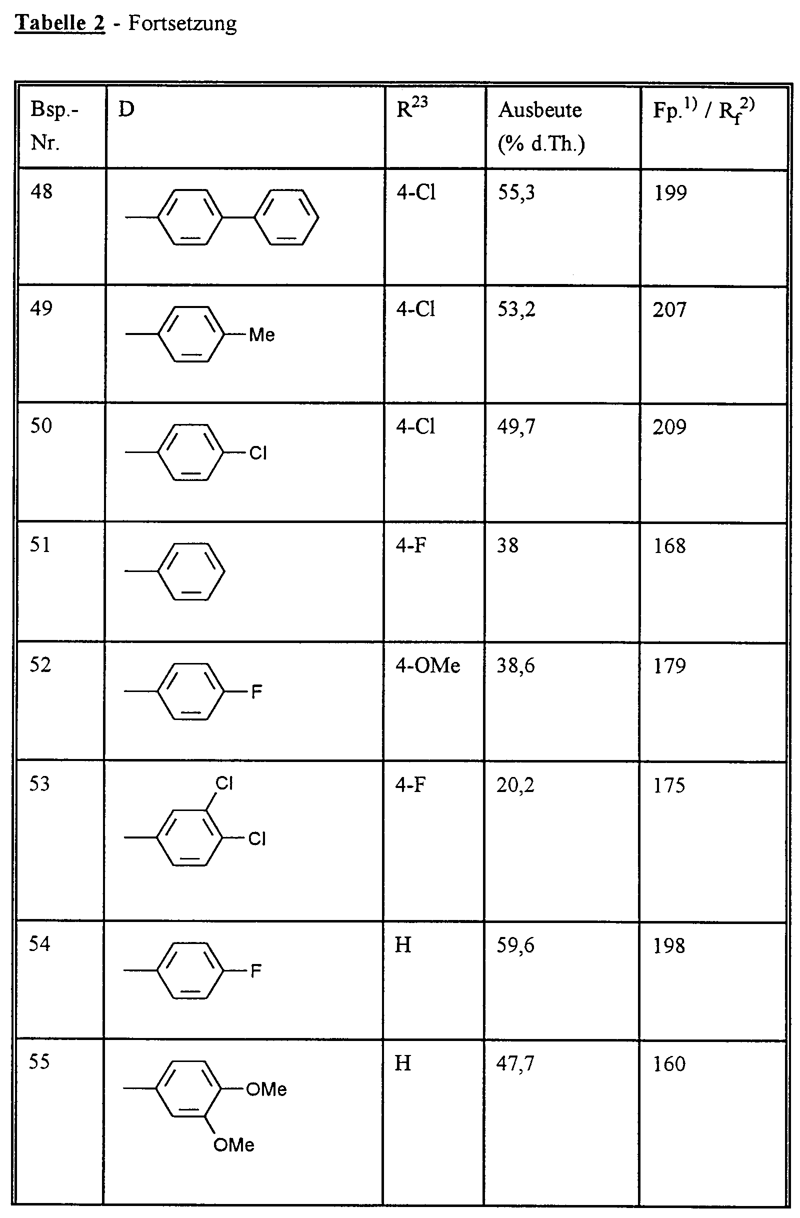

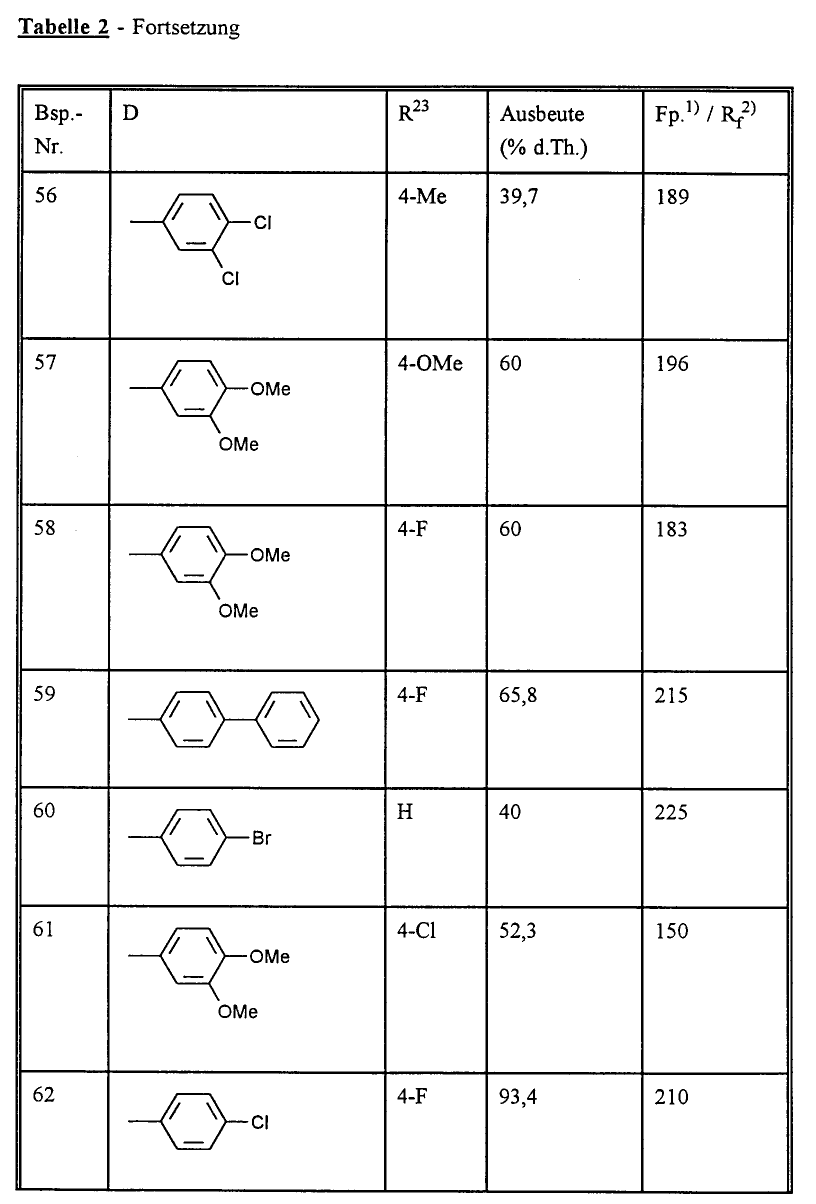

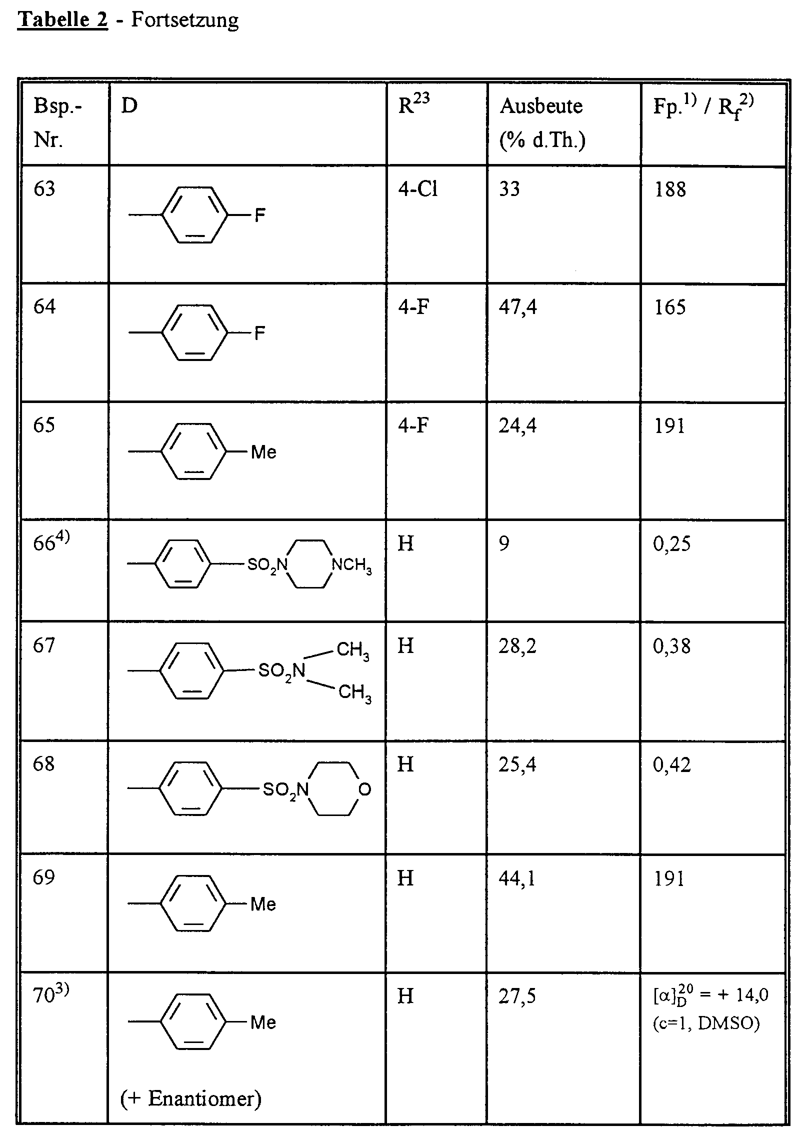

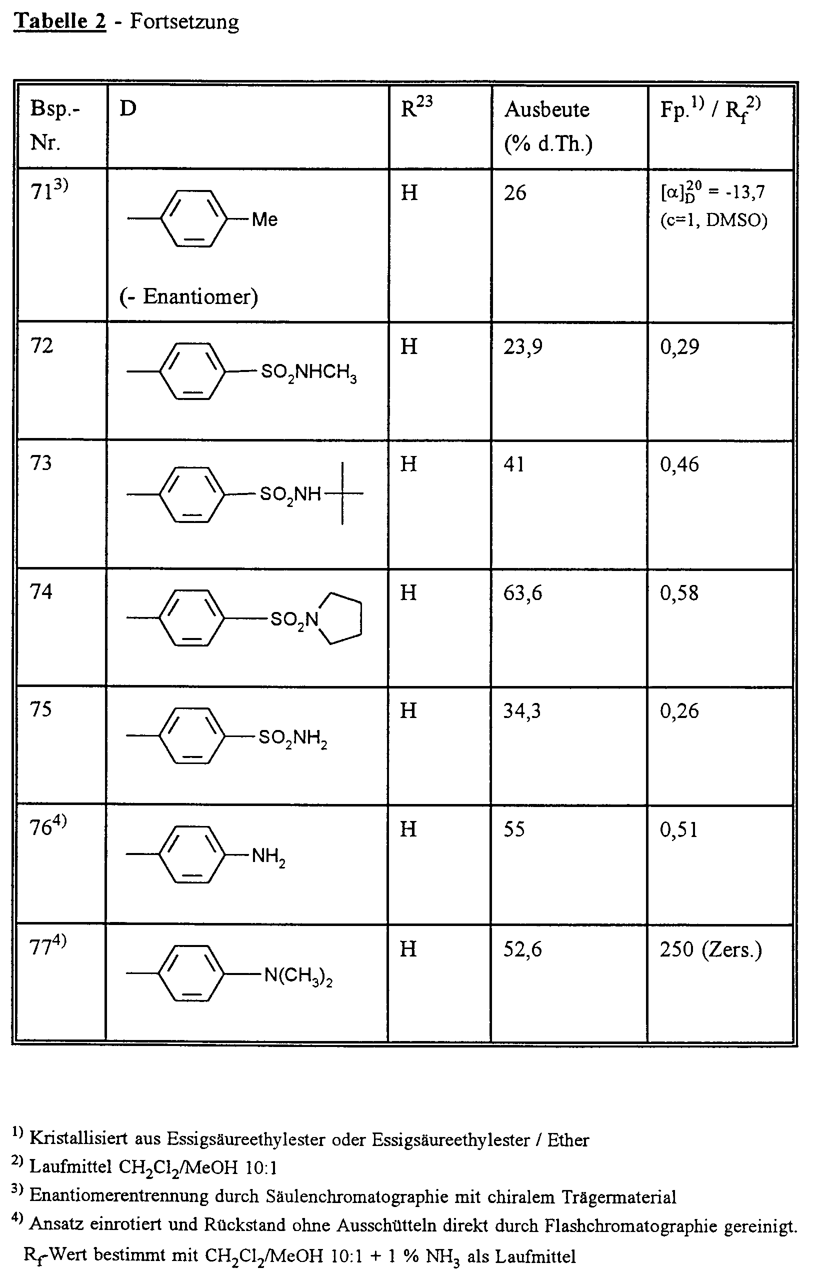

### Beispiel 78

### 9-(5-Phenyl-2-pentyl)-2-(4-p-methoxyphenylbenzyl)-purin-6-on

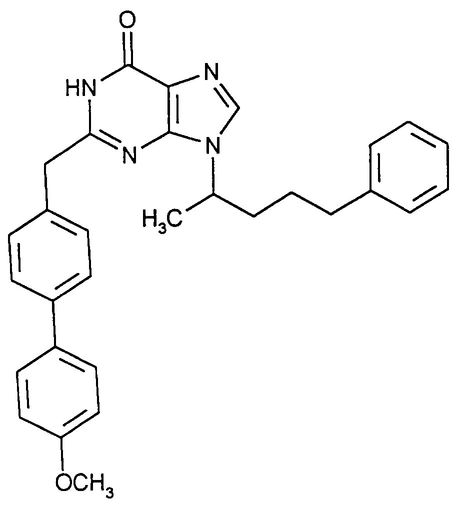

208 mg (0,46 mM) 9-(5-Phenyl-2-pentyl)-2-(4-brombenzyl)-purin-6-on (Beispiel 6), 90 mg (0,59 mM) p-Methoxyphenylboronsäure und 210 mg Bis(triphenylphosphin)-palladium-II-chlorid werden in 10 ml abs. THF 1 h bei 70°C gerührt. Danach werden 0,65 ml 2 N Na₂CO₃-Lösung zugegeben und es wird 5 h refluxiert (DC-Kontrolle). Der Ansatz wird eingedampft und der Rückstand durch Flashchromatographie (Kieselgel, Eluens CH₂Cl₂ / MeOH 30:1) gereinigt. Die das Produkt enthaltenden Fraktionen werden vereinigt und eingedampft. Der Rückstand wird aus Essigsäureethylester / Ether umkristallisiert.
Ausbeute: 161 mg (78,5%)
Fp.: 172°C

In analoger Weise wurden die in der Tabelle 3 aufgeführten Beispiele hergestellt:

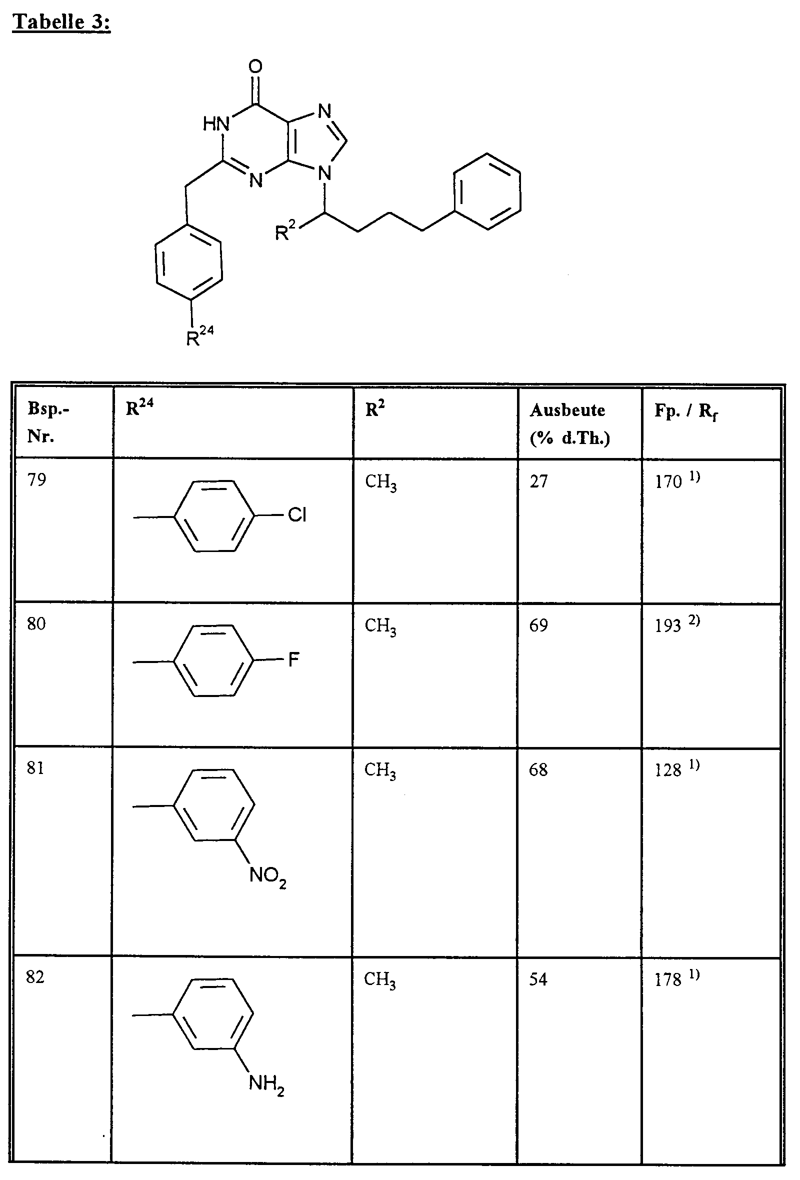

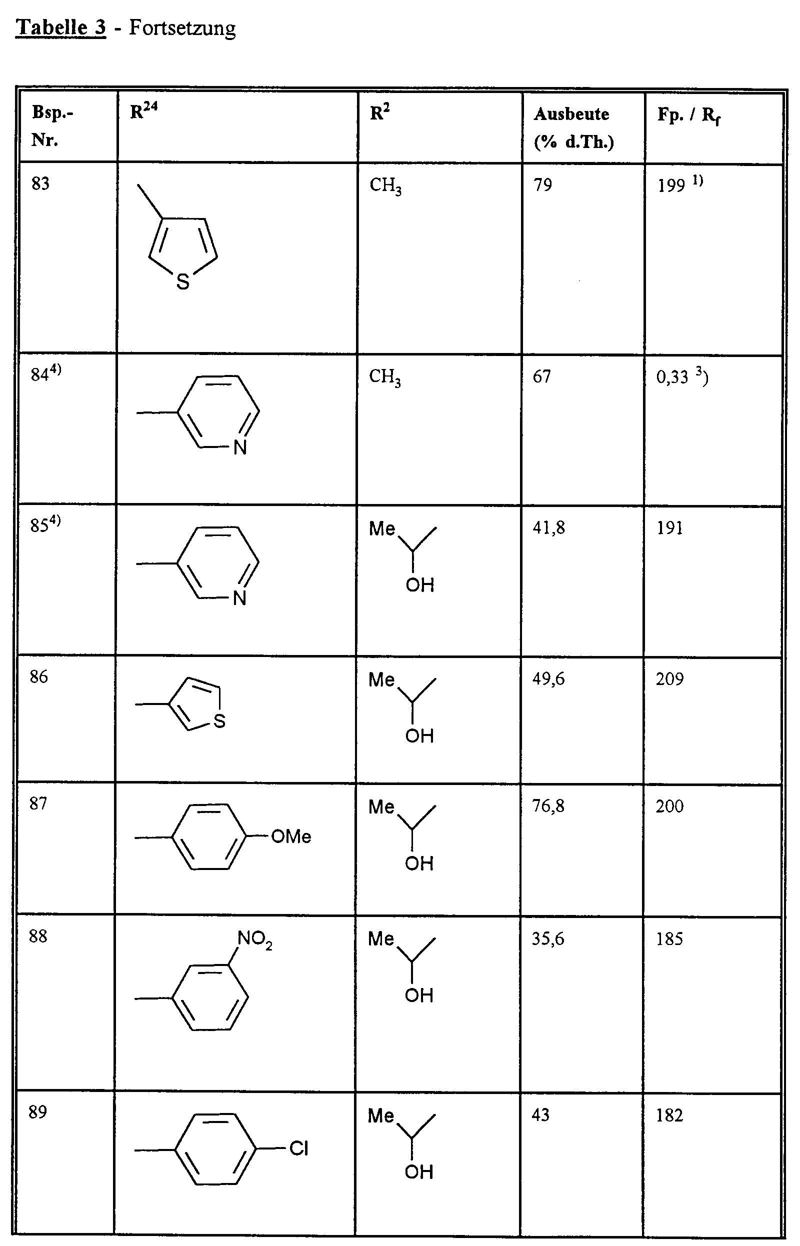

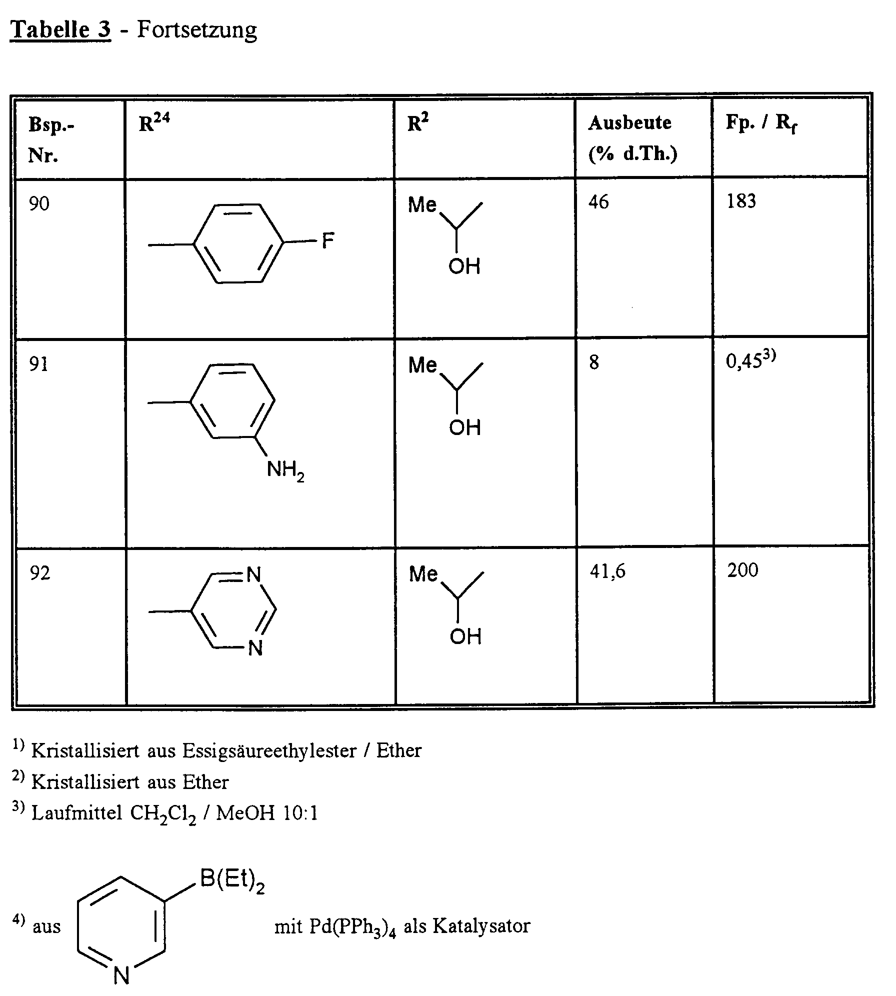

### Beispiel 93

### 9-(5-Phenyl-2-pentyl)-8-ethyl-2-benzyl-purin-6-on

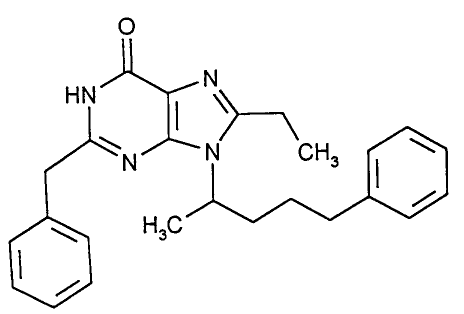

1,2 g (4 mmol) 1-(5-Phenyl-3-pentyl)-2-ethyl-5-amino-imidazol-4-carboxamid werden mit 240 mg (6 mmol) Natriumhydrid (60%) in 30 ml abs. THF und 10 ml abs. DMF 30 Minuten bei 50°C gerührt. Nach Abkühlen auf 20°C werden 773 mg (5 mmol) Phenylessigsäurechlorid in 10 ml abs.THF zugetropft und über Nacht bei 20°C gerührt. Langsam werden 10 ml H₂O, dann 10 ml 2 n HCl, dann 100 ml Essigsäurethylester zugegeben. Die organische Phase wird abgetrennt, über Na₂SO₄ getrocknet und eingedampft. Der Rückstand wird durch Flashchromatographie (Kieselgel, Eluens CH₂Cl₂ / MeOH 50:1 -> 20:1) gereinigt.
R_{f} = 0.32 (CH₂Cl₂ / MeOH 10:1)
Ausbeute: 441 mg (26,4%)
440 mg (1,05 mmol) des acylierten Imidazol-carboxamids und 0,58 g (4,2 mmol) Kaliumcarbonat werden in 20 ml Methanol und 5 ml H₂O über Nacht refluxiert. Nach dem Abkühlen werden 10 ml 2 n HCl zugegeben, 2 x mit je 20ml Essisäureethylester ausgeschüttelt und die vereinigten Essigesterphasen 1 x mit Wasser und 1 x mit ges. NaCl-Lösung gewaschen. Nach Trocknen über Na₂SO₄ wird eingedampft und der Rückstand durch Flashchromatographie (Kieselgel, Eluens CH₂Cl₂ / MeOH 30:1 -> 20:1) gereinigt.
Ausbeute: 161 mg (38,3%)
R_{f} = 0,33 (CH₂Cl₂ / MeOH 10:1)

In analoger Weise wurden die in der Tabelle 4 aufgeführten Beispiele hergestellt:

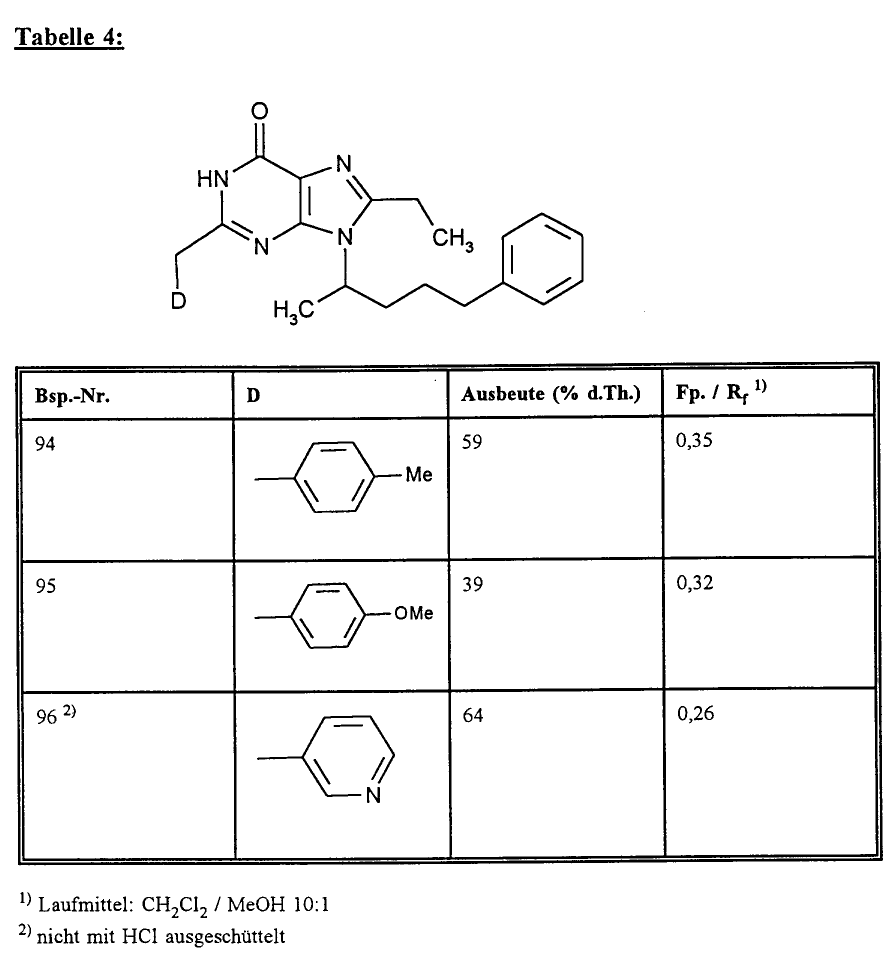

### Beispiel 97

### 9-(6-Phenyl-2-oxo-3-hexyl)-2-(4-phenyl-benzyl)-purin-6-on

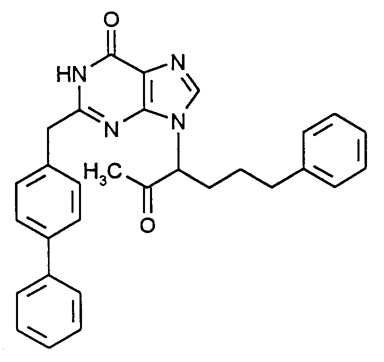

478 mg (1 mmol) 9-(6-Phenyl-2-hydroxy-3-hexyl)-2-(4-phenyl-benzyl)-purin-6-on (Bsp. 46) und 1,3 ml Triethylamin werden in 10 ml CH₂Cl₂ und 3 ml DMSO gelöst und bei 0°C werden 0,7 g Pyridin/SO₃-Komplex zugesetzt. Es wird 16 h bei 20°C gerührt und je einmal mit 10 ml 2 n HCl und 10 ml gesättigter NaHCO₃-Lösung ausgeschüttelt. Nach Trocknen der organischen Phase über Na₂SO₄ wird eingedampft und der Rückstand durch Flashchromatographie über Kieselgel (Eluens: CH₂Cl₂/MeOH 40:1) gereinigt. Ausbeute: 425 mg (89,3 %), R_{f} = 0,52 (CH₂Cl₂/MeOH 30:1)

In analoger Weise werden die in Tabelle 5 aufgeführten Verbindungen hergestellt.

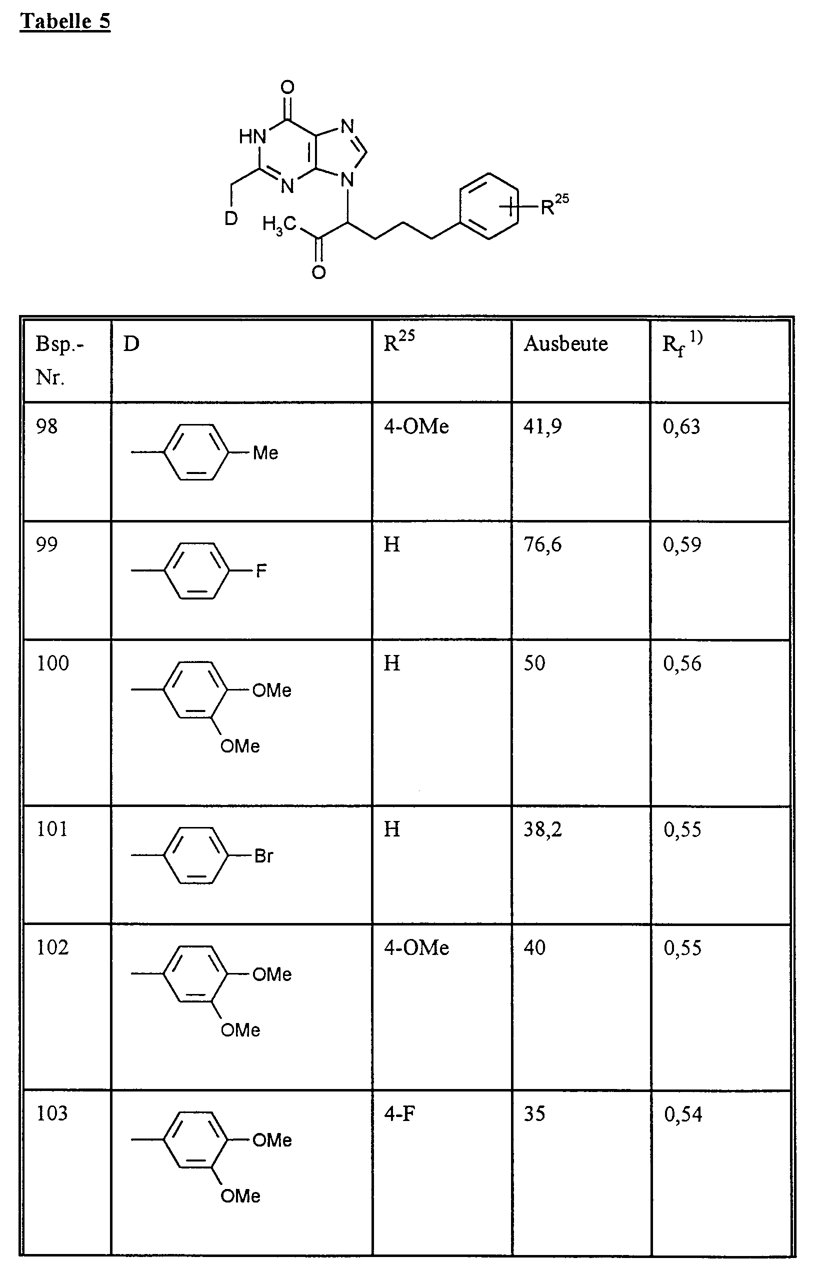

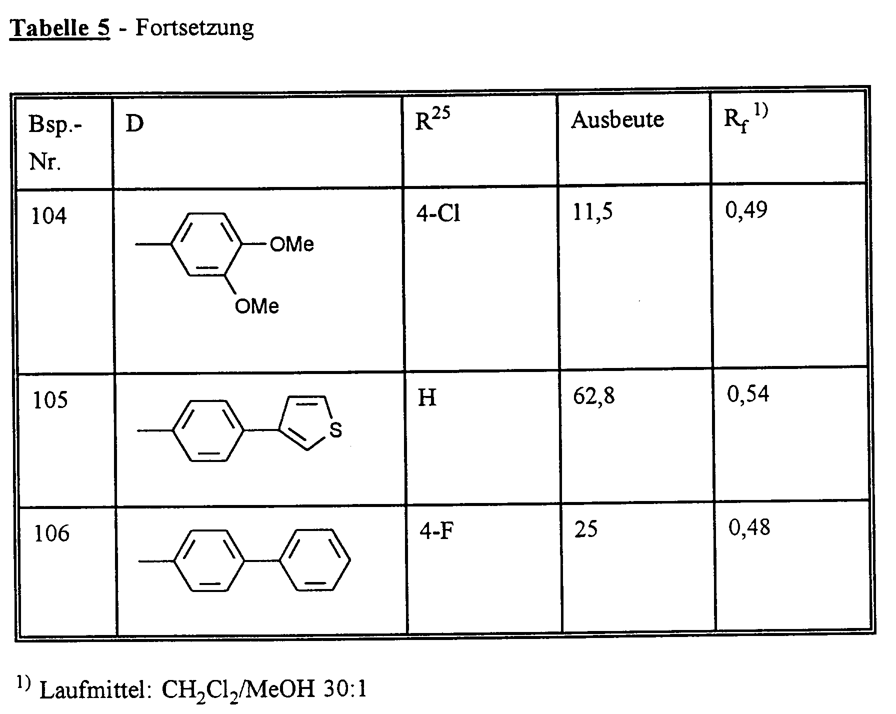

### Beispiel 107

### 9-(5-Phenyl-2-pentyl)-2-(4-hydroxybenzyl)-purin-6-on

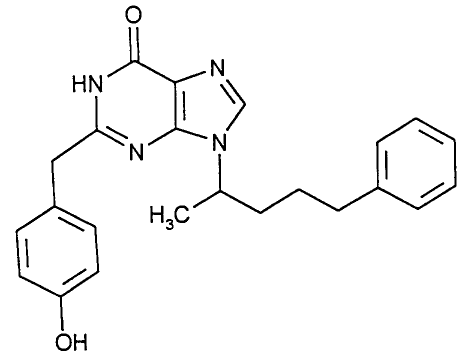

Zu 404 mg (1 mmol) der Verbindung aus Beispiel 7 in 5 ml abs. CH₂Cl₂ werden bei -78°C 2,2 ml einer 1 molaren Lösung von Bortribromid in CH₂Cl₂ getropft. Nach 30 Minuten bei -78°C wird 2 Stunden bei 20°C gerührt. Da im Dünnschichtchromatogramm noch Ausgangssubstanz zu sehen war, wurden weitere 1,1 ml der 1 molaren BBr₃-Lösung bei -78°C zugetropft. Nach Rühren über Nacht bei Raumtemperatur wird auf -78°C gekühlt, und es werden 5 ml Methanol zugetropft. Der Ansatz wird eingedampft, der Rückstand in 10 ml CH₂Cl₂ aufgenommen und 2 x mit je 10 ml 10% NaOH-Lösung ausgeschüttelt. Die vereinigten NaOH-Phasen werden durch Zugabe von konz. HCl-Lösung auf pH 1-2 angesäuert und 2 mal mit je 30 ml Essigsäureethylester ausgeschüttelt. Die vereinigten Essigesterphasen werden eingedampft und der Rückstand durch Flashchromatographie (Kieselgel, Eluens CH₂Cl₂ / MeOH 50:1 → 30:1) gereinigt. Umkristallisation aus Essigsäureethylester / Ether.
Ausbeute: 250 mg (64,4%)
Fp.: 184°C

### Beispiel 108

### 9-(1-Benzyloxy-2-propyl)-2-benzyl-purin-6-on

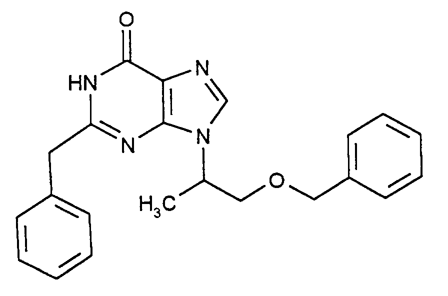

a) 22 g (0,22 mol) 2-Amino-2-cyano-acetamid und 37 ml Triethylorthoformiat werden 1 Stunde in 200 ml Acetonitril refluxiert. Dann werden 17,5 ml 2-Amino-1-propanol zugegeben und weitere 15 Minuten refluxiert. Nach dem Abkühlen wird eingeengt, der Rückstand in wenig Methanol gelöst und durch Flashchromatographie über Kieselgel (Eluens CH₂Cl₂/MeOH 10:1) gereinigt. Die Ausbeute an kristallinem 5-Amino-1-(1-hydroxy-2-propyl)-imidazol-4-carboxamid beträgt 24,3 g (60 %).
b) 24 g (0,13 mol) der Verbindung aus Schritt a) werden mit 25 ml Acetanhydrid und 30 ml Pyridin in 100 ml als CH₂Cl₂ 2 Stunden bei 50°C gerührt. Nach dem Abkühlen wird mit ges. NaCl ausgeschüttelt, die organische Phase über Na₂SO₄ getrocknet und eingedampft. Der Rückstand wird aus Essigsäureethylester/Ether kristallisiert. Die Ausbeute an 5-Amino-1-(1-acetoxy-2-propyl)-imidazol-4-carboxamid beträgt 23,4 g (84,2 %).
c) 23 g (0,107 mol) der Verbindung aus Schritt b) und 1 g DMAP (Dimethylaminopyridin) werden in 100 ml Pyridin gelöst. Zu dieser Lösung wird eine Lösung von 12,1 ml Phenylessigsäurechlorid in 20 ml abs. Toluol zugetropft und 30 Minuten bei 20°C gerührt. Danach wird 2 Stunden bei 60°C gerührt, eingedampft, 100 ml abs. Toluol zugegeben und wieder eingedampft. Der Rückstand wird in Methylenchlorid gelöst und die organische Phase mit Wasser gewaschen. Das Produkt kristallisiert aus der Methylenchloridphase aus, wird abgesaugt, getrocknet und ohne weitere Reinigung direkt weiter umgesetzt. Die Ausbeute an 5-Phenylacetylamino-1-(1-acetoxy-2-propyl)-imidazol-4-carboxamid beträgt 12,8 g (36 %).
d) 12,8 g (38,5 mmol) der Verbindung aus Schritt c) und 6,2 g (155 mmol) NaOH werden in 100 ml eines Methanol/Wasser-Gemisches (1:1) 3 Stunden refluxiert. Nach dem Abkühlen wird mit 10 % HCl angesäuert und 2 mal mit Essigsäureethylester extrahiert. Die vereinigten Essigsäureethylesterphasen werden mit gesättigter NaCl-Lösung gewaschen, über Na₂SO₄ getrocknet und eingedampft. Der Rückstand wird aus Essigsäureethylester/Ether umkristallisiert. Die Ausbeute an 9-(1-Hydroxy-2-propyl)-2-benzyl-purin-6-on beträgt 6,2 g (55,2 %), Fp.: 192°C.
e) 576 mg (2 mmol) des Purin-6-ons aus Schritt d) und 200 mg NaH (60%ig) werden in 30 ml abs. THF 30 Minuten refluxiert. Nach dem Abkühlen werden bei 20°C 0,3 ml (2,4 mmol) Benzylchlorid in wenig abs. THF zugetropft und 16 Stunden gerührt. Es werden 5 ml H₂O zugetropft, dann wird eingedampft und der Rückstand in Essigsäureethylester aufgenommen. Die organische Phase wird mit 2 n HCl und gesättigter NaCl-Lösung gewaschen, über Na₂SO₄ getrocknet und eingedampft. Der Rückstand wird durch Flashchromatographie über Kieselgel (Eluens: CH₂Cl₂/MeOH 40:1) gereinigt. Die das Produkt enthaltenden Fraktionen werden vereinigt und eingedampft. Der Rückstand wird mit Diethylether kristallisiert. Ausbeute 510 mg (67,5 %), Fp. 149°C.

### Beispiel 109

### 9-(1-p-Chlorbenzyloxy-2-propyl)-2-benzyl-purin-6-on

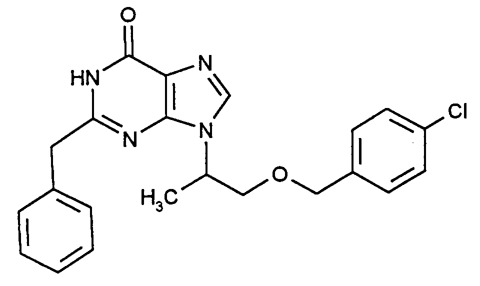

Die Titelverbindung wurde in analoger Weise zur Vorschrift des Beispiels 108 hergestellt. Ausbeute: 59 %, Fp. 166°C.

Die in Tabelle 6 aufgeführten Beispiele wurden nach den oben aufgeführten Vorschriften in Analogie zu den Verbindungen aus der Tabelle 2 in der Regel nach Methode c) hergestellt.

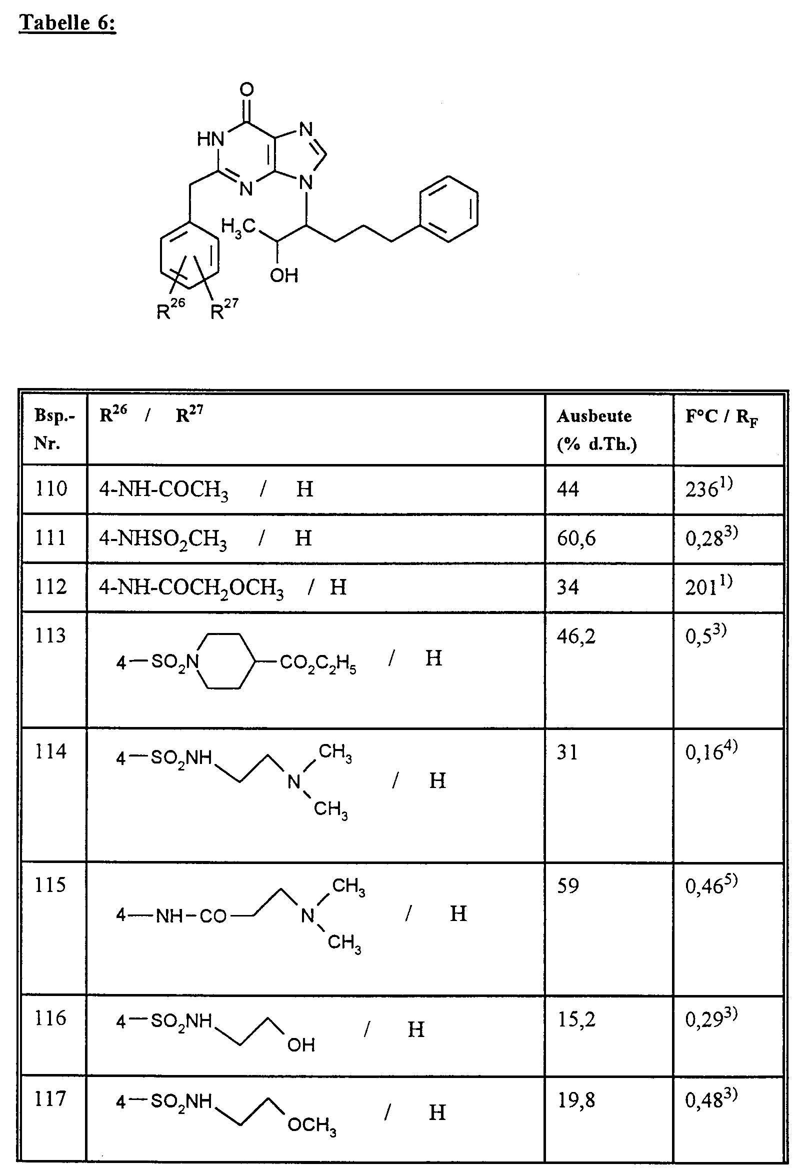

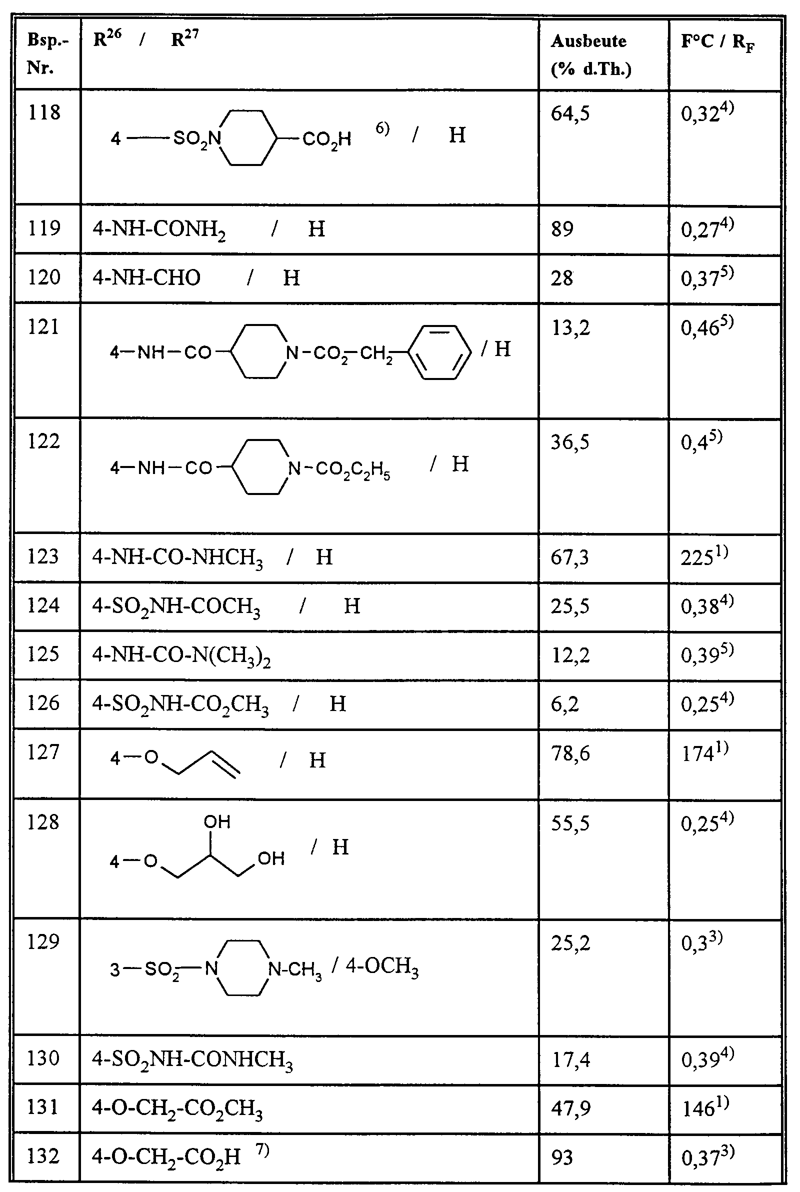

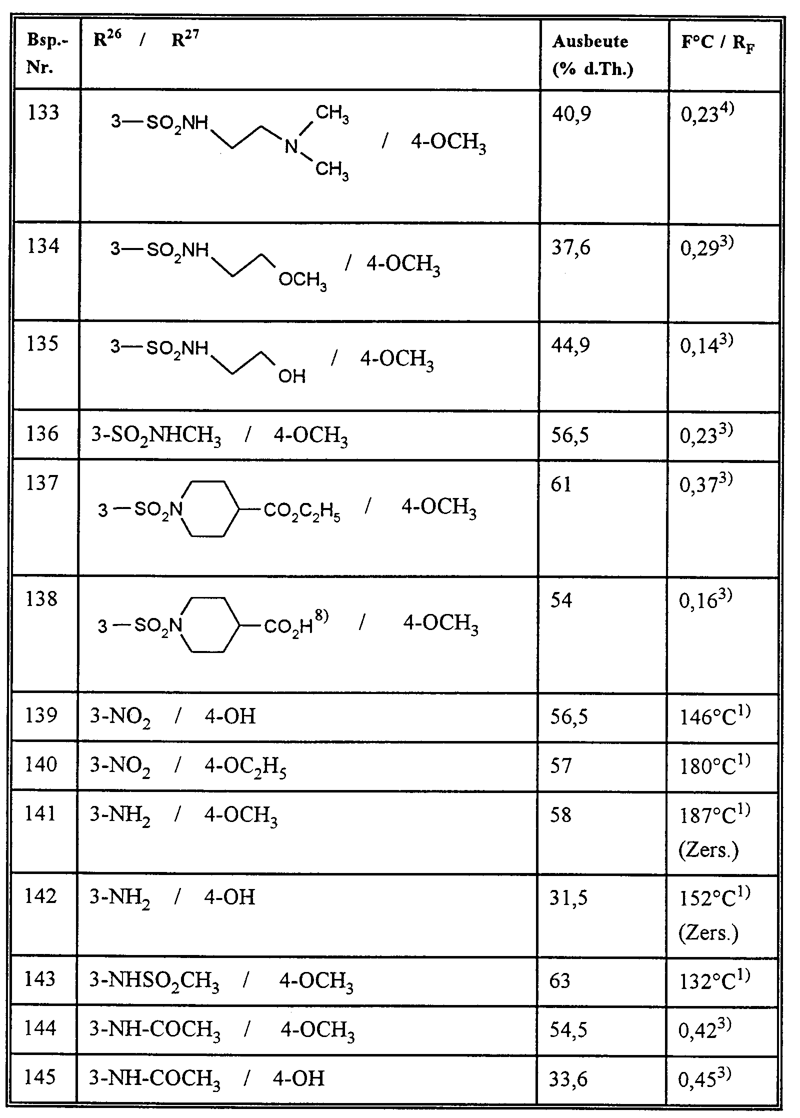

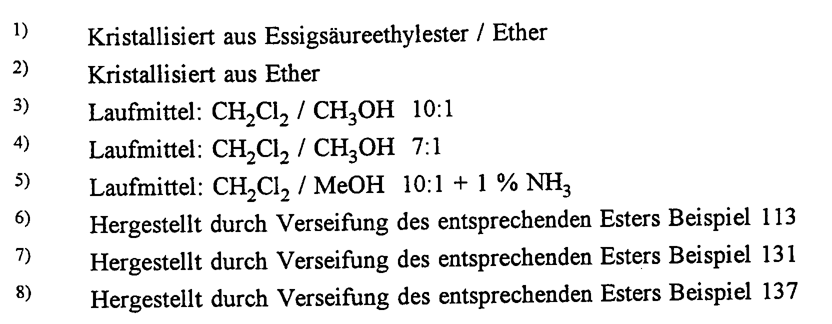

## Patentansprüche

1. Purin-6-on-derivate der allgemeinen Formel (I) 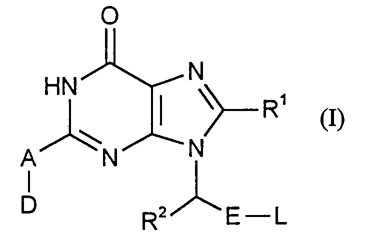 in welcher
R¹ für Wasserstoff oder für geradkettiges oder verzweigtes Alkyl mit bis zu 8 Kohlenstoffatomen steht,
R² für geradkettiges oder verzweigtes Acyl mit bis zu 4 Kohlenstoffatomen steht, oder
für geradkettiges oder verzweigtes Alkyl mit bis zu 8 Kohlenstoffatomen steht, das gegebenenfalls durch Hydroxy, Azido oder durch eine Gruppe der Formel -NR³R⁴ oder -OSO₂R⁵ substituiert ist,
worin
R³ und R⁴ gleich oder verschieden sind und
Cycloalkyl mit 3 bis 6 Kohlenstoffatomen, Wasserstoff, Formyl, oder geradkettiges oder verzweigtes Alkyl mit bis zu 6 Kohlenstoffatomen bedeuten, das gegebenenfalls durch Hydroxy, Carboxy oder durch geradkettiges oder verzweigtes Alkoxy oder Alkoxycarbonyl mit jeweils bis zu 6 Kohlenstoffatomen oder durch eine Gruppe der Formel -(CO)ₐ-NR⁶R⁷ substituiert ist,
worin
a eine Zahl 0 oder 1 bedeutet,
R⁶ und R⁷ gleich oder verschieden sind und Wasserstoff, Formyl, Hydroxy, Phenyl oder geradkettiges oder verzweigtes Alkyl mit bis zu 6 Kohlenstoffatomen bedeuten, das gegebenenfalls durch Hydroxy oder durch geradkettiges oder verzweigtes Alkoxy mit bis zu 5 Kohlenstoffatomen substituiert ist,
oder
R³ und/oder R⁴ geradkettiges oder verzeigtes Alkoxycarbonyl mit bis zu 6 Kohlenstoffatomen, Carboxy oder geradkettiges oder verzweigtes Acyl mit bis zu 6 Kohlenstoffatomen bedeuten, das gegebenenfalls durch Hydroxy oder geradkettiges oder verzweigtes Alkoxy mit bis zu 4 Kohlenstoffatomen substituiert ist
oder
R³ und/oder R⁴ einen Rest der Formel -(CO)_{b}-T-NR⁸R⁹, -CO-R¹⁰, -SO₂R¹¹ oder -SO₂NR¹²R¹³ bedeuten, worin
b die oben angegebene Bedeutung von a hat und mit dieser gleich oder verschieden ist
T geradkettiges oder verzweigtes Alkyl mit bis zu 5 Kohlenstoffatomen bedeutet oder im Fall b ≠ 0 auch eine Bindung bedeuten kann,
R⁸ und R⁹ die oben angegebene Bedeutung von R⁶ und R⁷ haben und mit dieser gleich oder verschieden sind
R¹⁰ einen gesättigten, partiell ungesättigten oder ungesättigten 5- bis 7-gliedrigen Heterocyclus mit bis zu 3 Heteroatomen aus der Reihe S, N und/oder O bedeutet, der gegebenenfalls auch über die N-Funktion, durch geradkettiges oder verzweigtes Alkyl, Alkoxy oder Alkoxycarbonyl mit jeweils bis zu 4 Kohlenstoffatomen, Carboxyl, Benzyloxycarbonyl oder Hydroxy substituiert sein kann,
R¹¹ geradkettiges oder verzweigtes Alkyl mit bis zu 5 Kohlenstoffatomen, Benzyl oder Phenyl bedeutet,
R¹² und R¹³ gleich oder verschieden sind und Wasserstoff, Phenyl oder geradkettiges oder verzweigtes Alkyl mit bis zu 6 Kohlenstoffatomen bedeuten,
oder
R³ und R⁴ gemeinsam mit dem Stickstoffatom einen 5- oder 6-gliedrigen gesättigten, partiell ungesättigten oder ungesättigten Heterocyclus bilden, der bis zu 3 Heteroatomen aus der Reihe N, S und/oder 0 oder einen Rest -NR¹⁴ enthalten kann, und der gegebenenfalls durch Carbonyl, durch geradkettiges oder verzweigtes Alkoxycarbonyl mit bis zu 5 Kohlenstoffatomen oder durch geradkettiges oder verzweigtes Alkyl mit bis zu 5 Kohlenstoffatomen substituiert ist, das seinerseits durch Hydroxy, Carboxy oder durch geradkettiges oder verzweigtes Acyl, Alkoxy oder Alkoxycarbonyl mit jeweils bis zu 6 Kohlenstoffatomen substituiert sein kann,
worin
R¹⁴ Wasserstoff, Carbonyl oder geradkettiges oder verzweigtes Alkyl oder Alkoxycarbonyl mit jeweils bis zu 5 Kohlenstoffatomen bedeutet,
und
R⁵ Phenyl oder geradkettiges oder verzweigtes Alkyl mit bis zu 5 Kohlenstoffatomen bedeutet,
A für eine geradkettige oder verzweigte Alkylen- oder Alkenylenkette mit jeweils bis zu 6 Kohlenstoffatomen steht,
D und L gleich oder verschieden sind und für Aryl mit 6 bis 10 Kohlenstoffatomen oder für einen 5- bis 7-gliedrigen aromatischen, gegebenenfalls benzokondensiertenHeterocyclus mit bis zu 3 Heteroatomen aus der Reihe S, N und/oder O stehen, die gegebenenfalls bis zu 3-fach gleich oder verschieden durch Halogen, Hydroxy, Nitro, Trifluormethyl, Carboxy, geradkettiges oder verzweigtes Alkyl, Alkoxy oder Alkoxycarbonyl mit jeweils bis zu 6 Kohlenstoffatomen oder durch eine Gruppe der Formel -(V)_{c}-NR¹⁵R¹⁶ und/oder -OR¹⁷ substituiert sind,
worin
c eine Zahl 0 oder 1 bedeutet,
V einen Rest der Formel -CO oder -SO₂ bedeutet,
R¹⁵ und R¹⁶ gleich oder verschieden sind und die oben angegebene Bedeutung von R³ und R⁴ haben,
R¹⁷ Wasserstoff, geradkettiges oder verzweigtes Alkenyl mit bis zu 8 Kohlenstoffatomen oder geradkettiges oder verzweigtes Alkyl mit bis zu 8 Kohlenstoffatomen bedeutet, das gegebenenfalls bis zu 3-fach gleich oder verschieden durch Hydroxy, Carboxyl oder geradkettiges oder verzweigtes Alkoxycarbonyl mit bis zu 5 Kohlenstoffatomen substituiert ist,
und/oder die Cyclen gegebenenfalls durch Aryl mit 6 bis 10 Kohlenstoffatomen oder durch einen 5- bis 7-gliedrigen aromatischen, gegebenenfalls benzokondensierten Heterocyclus mit bis zu 3 Heteroatomen aus der Reihe S, N und/oder O substituiert sind, die ihrerseits gegebenenfalls bis zu 2-fach gleich oder verschieden durch Halogen, Hydroxy, Nitro, Carboxyl, Trifluormethyl oder durch geradkettiges oder verzweigtes Alkyl, Alkoxy oder Alkoxycarbonyl mit jeweils bis zu 5 Kohlenstoffatomen oder durch eine Gruppe der Formel (V')_{d} -NR¹⁸R¹⁹ substituiert sind,
worin
d die oben angegebene Bedeutung von a hat und mit dieser gleich oder verschieden ist,
R¹⁸ und R¹⁹ die oben angegebene Bedeutung von R³ und R⁴ haben und mit dieser gleich oder verschieden sind,
V' die oben angegebene Bedeutung von V hat und mit dieser gleich oder verschieden ist,
und/oder
die unter D aufgeführten Ringsysteme
gegebenenfalls durch geradkettiges oder verzweigtes Acyl mit bis zu 5 Kohlenstoffatomen substituiert sind, das gegebenenfalls durch Hydroxy, geradkettiges oder verzweigtes Alkoxy mit bis zu 5 Kohlenstoffatomen oder durch eine Gruppe der Formel -NR²⁰R²¹ substituiert ist,
worin
R²⁰ und R²¹ gleich oder verschieden und die oben angegebene Bedeutung von R³ und R⁴ haben,
oder
D für einen Rest der Formel 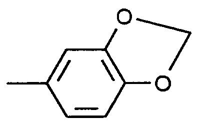 steht
und
E für einen Rest der Formel -CH₂-Y-Z- steht,
worin
Y eine Bindung oder ein Sauerstoff- oder Schwefelatom oder die -NH-Gruppe bedeutet,
Z eine geradkettige oder verzweigte Alkylenkette mit bis zu 5 Kohlenstoffatomen bedeutet,
und deren Tautomere und Salze.

2. Purin-6-on-derivate der Formel nach Anspruch 1, in welcher
R¹ für Wasserstoff oder für geradkettiges oder verzweigtes Alkyl mit bis zu 6 Kohlenstoffatomen steht,
R² für geradkettiges oder verzweigtes Acyl mit bis zu 6 Kohlenstoffatomen steht, oder
für geradkettiges oder verzweigtes Alkyl mit bis zu 6 Kohlenstoffatomen steht, das gegebenenfalls durch Hydroxy, Azido oder durch eine Gruppe der Formel -NR³R⁴ oder O-SO₂-R⁵ substituiert ist,
worin
R³ und R⁴ gleich oder verschieden sind und
Cyclopentyl, Cyclohexyl, Wasserstoff, Formyl oder geradkettiges oder verzweigtes Alkyl mit bis zu 5 Kohlenstoffatomen bedeuten, das gegebenenfalls durch Hydroxy, Carboxy oder durch geradkettiges oder verzweigtes Alkoxy oder Alkoxycarbonyl mit jeweils bis zu 5 Kohlenstoffatomen oder durch eine Gruppe der Formel -(CO)ₐ-NR⁶R⁷ substituiert ist,
worin
a eine Zahl 0 oder 1 bedeutet,
R⁶ und R⁷ gleich oder verschieden sind und Wasserstoff, Formyl, Hydroxy, Phenyl oder geradkettiges oder verzweigtes Alkyl mit bis zu 5 Kohlenstoffatomen bedeuten, das gegebenenfalls durch Hydroxy oder durch geradkettiges oder verzweigtes Alkoxy mit bis zu 4 Kohlenstoffatomen substituiert ist,
oder
R³ und/oder R⁴ geradkettiges oder verzeigtes Alkoxycarbonyl mit bis zu 5 Kohlenstoffatomen, Carboxy oder geradkettiges oder verzweigtes Acyl mit bis zu 5 Kohlenstoffatomen bedeuten, das gegebenenfalls durch Hydroxy oder geradkettiges oder verzweigtes Alkoxy mit bis zu 3 Kohlenstoffatomen substituiert ist
oder
R³ und/oder R⁴ einen Rest der Formel -(CO)_{b}-T-NR⁸R⁹, -CO-R¹⁰, -SO₂R¹¹ oder -SO₂NR¹²R¹³ bedeuten, worin
b die oben angegebene Bedeutung von a hat und mit dieser gleich oder verschieden ist
T geradkettiges oder verzweigtes Alkyl mit bis zu 4 Kohlenstoffatomen bedeutet
oder im Fall b ≠ 0 auch eine Bindung bedeuten kann,
R⁸ und R⁹ die oben angegebene Bedeutung von R⁶ und R⁷ haben und mit dieser gleich oder verschieden sind
R¹⁰ Morpholinyl, Imidazolyl, Pyridyl, Piperazinyl, Piperidinyl oder Pyrrolidinyl bedeutet, die gegebenenfalls, auch über die N-Funktion, durch geradkettiges oder verzweigtes Alkyl, Alkoxy oder Alkoxycarbonyl mit jeweils bis zu 3 Kohlenstoffatomen, Carboxyl, Benzyloxycarbonyl oder Hydroxy substituiert sein können,
R¹¹ geradkettiges oder verzweigtes Alkyl mit bis zu 4 Kohlenstoffatomen, Benzyl oder Phenyl bedeutet,
R¹² und R¹³ gleich oder verschieden sind und Wasserstoff, Phenyl oder geradkettiges oder verzweigtes Alkyl mit bis zu 5 Kohlenstoffatomen bedeuten,
oder
R³ und R⁴ gemeinsam mit dem Stickstoffatom einen Pyrrolidinyl-, Morpholinyl-, Imidazolyl-, Piperidinyl-, Piperazinylring bilden, die, gegebenenfalls auch über die Stickstofffunktion, durch geradkettiges oder verzweigtes Alkoxycarbonyl mit bis zu 4 Kohlenstoffatomen, Carboxyl oder durch geradkettiges oder verzweigtes Alkyl mit bis zu 4 Kohlenstoffatomen substituiert sein können, das seinerseits durch Carboxy, Hydroxy oder durch geradkettiges oder verzweigtes Acyl, Alkoxy oder Alkoxycarbonyl mit jeweils bis zu 4 Kohlenstoffatomen substituiert sein kann,
und
R⁵ Phenyl oder geradkettiges oder verzweigtes Alkyl mit bis zu 4 Kohlenstoffatomen bedeutet,
A für eine geradkettige oder verzweigte Alkylen- oder Alkenylenkette mit jeweils bis zu 5 Kohlenstoffatomen steht,
D und L gleich oder verschieden sind und für Phenyl, Naphthyl, Pyridyl, Pyrimidyl, Thienyl, Indolyl oder Furyl stehen, die gegebenenfalls bis zu 3-fach gleich oder verschieden durch Fluor, Chlor, Brom, Trifluormethyl, Nitro, Carboxy, geradkettiges oder verzweigtes Alkyl oder Alkoxycarbonyl mit jeweils bis zu 5 Kohlenstoffatomen oder durch eine Gruppe der Formel -(V)_{c}NR¹⁵R¹⁶ und/oder -OR¹⁷ substituiert sind,
worin
c eine Zahl 0 oder 1 bedeutet,
V einen Rest der Formel -CO oder -SO₂ bedeutet,
R¹⁵ und R¹⁶ gleich oder verschieden sind und die oben angegebene Bedeutung von R³ und R⁴ haben,
R¹⁷ Wasserstoff, geradkettiges oder verzweigtes Alkenyl mit bis zu 6 Kohlenstoffatomen oder geradkettiges oder verzweigtes Alkyl mit bis zu 6 Kohlenstoffatomen bedeutet, das gegebenenfalls bis zu 2-fach gleich oder verschieden durch Hydroxy, Carboxyl oder durch geradkettiges oder verzweigtes Alkoxycarbonyl mit bis zu 4 Kohlenstoffatomen substituiert ist,
und/oder die Cyclen gegebenenfalls durch Naphthyl, Phenyl, Pyridyl, Indolyl, Thienyl, Furyl, Pyridazinyl, Pyridyl, Pyrryl oder Pyrimidyl substituiert sind, die ihrerseits gegebenenfalls durch Fluor, Chlor, Brom, Hydroxy, Nitro, Carboxyl, Trifluormethyl oder durch geradkettiges oder verzweigtes Alkyl, Alkoxy oder Alkoxycarbonyl mit jeweils bis zu 3 Kohlenstoffatomen oder durch eine Gruppe der Formel -(V')_{d}NR¹⁸R¹⁹ substituiert sind,
worin
d die oben angegebene Bedeutung von a hat und mit dieser gleich oder verschieden ist,
V' die oben angegebene Bedeutung von V hat und mit dieser gleich oder verschieden ist,
R¹⁸ und R¹⁹ die oben angegebene Bedeutung von R³ und R⁴ haben,
und/oder
die unter D aufgeführten Ringsysteme
gegebenenfalls durch geradkettiges oder verzweigtes Acyl mit bis zu 4 Kohlenstoffatomen substituiert ist, das gegebenenfalls durch Hydroxy, geradkettiges oder verzweigtes Alkoxy mit bis zu 4 Kohlenstoffatomen oder durch eine Gruppe der Formel -NR²⁰R²¹ substituiert ist,
worin
R²⁰ und R²¹ gleich oder verschieden sind und die oben angegebene Bedeutung von R³ und R⁴ haben,
oder D für einen Rest der Formel 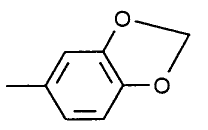 steht,
E für einen Rest der Formel -CH₂-Y-Z- steht,
worin
Y eine Bindung oder ein Sauerstoff- oder Schwefelatom oder die -NH-Gruppe bedeutet,
Z eine geradkettige oder verzweigte Alkylenkette mit bis zu 4 Kohlenstoffatomen bedeutet,
und deren Tautomere und Salze.

3. Purin-6-on-derivate der Formel nach Anspruch 1, in welcher
R¹ für Wasserstoff oder für geradkettiges oder verzweigtes Alkyl mit bis zu 4 Kohlenstoffatomen steht,
R² für geradkettiges oder verzweigtes Acyl mit bis zu 5 Kohlenstoffatomen steht, oder
für geradkettiges oder verzweigtes Alkyl mit bis zu 5 Kohlenstoffatomen steht, das gegebenenfalls durch Hydroxy, Azido oder durch eine Gruppe der Formel-NR³R⁴ oder O-SO₂R⁵ substituiert ist,
worin
R³ und R⁴ gleich oder verschieden sind und
Cyclopentyl, Wasserstoff, Formyl oder geradkettiges oder verzweigtes Alkyl mit bis zu 4 Kohlenstoffatomen bedeuten, das gegebenenfalls durch Hydroxy, Carboxy oder durch geradkettiges oder verzweigtes Alkoxy oder Alkoxycarbonyl mit jeweils bis zu 3 Kohlenstoffatomen oder durch eine Gruppe der Formel -(CO)ₐ-NR⁶R⁷ substituiert ist,
worin
a eine Zahl 0 oder 1 bedeutet,
R⁶ und R⁷ gleich oder verschieden sind und Wasserstoff, Formyl, Hydroxy oder geradkettiges oder verzweigtes Alkyl mit bis zu 3 Kohlenstoffatomen bedeuten, das gegebenenfalls durch Hydroxy oder durch geradkettiges oder verzweigtes Alkoxy mit bis zu 3 Kohlenstoffatomen substituiert ist,
oder
R³ und/oder R⁴ geradkettiges oder verzeigtes Alkoxycarbonyl mit bis zu 3 Kohlenstoffatomen, Carboxy oder geradkettiges oder verzweigtes Acyl mit bis zu 3 Kohlenstoffatomen bedeuten, das gegebenenfalls durch Hydroxy, Methoxy oder Ethoxy substituiert ist
oder
R³ und/oder R⁴ einen Rest der Formel -(CO)_{b}-T-NR⁸R⁹, -CO-R¹⁰, -SO₂R¹¹ oder -SO₂NR¹²R¹³ bedeuten, worin
b die oben angegebene Bedeutung von a hat und mit dieser gleich oder verschieden ist
T geradkettiges oder verzweigtes Alkyl mit bis zu 3 Kohlenstoffatomen bedeutet oder im Fall b ≠ 0 auch eine Bindung bedeuten kann,
R⁸ und R⁹ die oben angegebene Bedeutung von R⁶ und R⁷ haben und mit dieser gleich oder verschieden sind
R¹⁰ Morpholinyl, Piperidinyl, Piperazinyl oder Pyrrolidinyl bedeutet, die, gegebenenfalls auch über die N-Funktion, durch geradkettiges oder verzweigtes Alkyl, Alkoxy oder Alkoxycarbonyl mit jeweils bis zu 3 Kohlenstoffatomen, Benzyloxycarbonyl, Carboxyl oder Hydroxy substituiert sein können,
R¹¹ geradkettiges oder verzweigtes Alkyl mit bis zu 3 Kohlenstoffatomen, Benzyl oder Phenyl bedeutet,
R¹² und R¹³ gleich oder verschieden sind und Wasserstoff oder geradkettiges oder verzweigtes Alkyl mit bis zu 4 Kohlenstoffatomen bedeuten,
oder
R³ und R⁴ gemeinsam mit dem Stickstoffatom einen Pyrrolidinyl-, Morpholinyl-, Imidazolyl, Piperidinyl- oder Piperazinylring bilden, die, gegebenenfalls auch über die Stickstofffunktion, durch geradkettiges oder verzweigtes Alkyl oder Alkoxycarbonyl mit jeweils bis zu 3 Kohlenstoffatomen oder Carboxyl oder durch geradkettiges oder verzweigtes Alkyl mit bis zu 3 Kohlenstoffatomen substituiert sein können, das seinerseits durch Carboxy, Hydroxy oder durch geradkettiges oder verzweigtes Acyl, Alkoxy oder Alkoxycarbonyl mit jeweils bis zu 3 Kohlenstoffatomen substituiert sein kann
und
R⁵ Phenyl oder geradkettiges oder verzweigtes Alkyl mit bis zu 3 Kohlenstoffatomen bedeutet,
A für eine Ethenyl (-Vinyl) oder eine Alkylenkette mit bis zu 4 Kohlenstoffatomen steht,
worin
D und L gleich oder verschieden sind und für Phenyl, Naphthyl, Furyl, Thienyl, Indolyl oder Pyridyl stehen, die gegebenenfalls bis zu 2-fach gleich oder verschieden durch Fluor, Chlor, Brom, Nitro, Carboxy, Trifluormethyl, geradkettiges oder verzweigtes Alkyl oder Alkoxycarbonyl mit jeweils bis zu 4 Kohlenstoffatomen oder durch eine Gruppe der Formel -(V)_{c}NR¹⁵R¹⁶ und/oder -OR¹⁷ substituiert sind,
worin
c eine Zahl 0 oder 1 bedeutet,
V einen Rest der Formel -CO oder -SO₂ bedeutet,
R¹⁵ und R¹⁶ gleich oder verschieden sind und die oben angegebene Bedeutung von R³ und R⁴ haben,
R¹⁷ Wasserstoff, geradkettiges oder verzweigtes Alkenyl mit bis zu 4 Kohlenstoffatomen oder geradkettiges oder verzweigtes Alkyl mit bis zu 4 Kohlenstoffatomen bedeutet, das gegebenenfalls bis zu 2-fach gleich oder verschieden durch Hydroxyl, Carboxyl oder durch geradkettiges oder verzweigtes Alkoxycarbonyl mit bis zu 3 Kohlenstoffatomen substituiert ist
und/oder die Cyclen gegebenenfalls durch Phenyl, Pyrimidyl, Pyridazinyl, Pyridyl, Thienyl oder Furyl substituiert sind, die ihrerseits gegebenenfalls durch Fluor, Chlor, Brom, Hydroxy, Nitro, Carboxyl, Trifluormethyl oder durch geradkettiges oder verzweigtes Alkyl, Alkoxy oder Alkoxycarbonyl mit jeweils bis zu 3 Kohlenstoffatomen oder durch eine Gruppe der Formel -(V')_{d}NR¹⁸R¹⁹ substituiert sind,
worin
d die oben angegebene Bedeutung von a hat und mit dieser gleich oder verschieden ist,
V' die oben angegebene Bedeutung von V hat und mit dieser gleich oder verschieden ist,
R¹⁸ und R¹⁹ die oben angegebene Bedeutung von R³ und R⁴ haben,
und/oder
D gegebenenfalls durch geradkettiges oder verzweigtes Acyl mit bis zu 3 Kohlenstoffatomen substituiert ist, das gegebenenfalls durch Hydroxy, geradkettiges oder verzweigtes Alkoxy mit bis zu 3 Kohlenstoffatomen oder durch eine Gruppe der Formel -NR²⁰R²¹ substituiert ist,
worin
R²⁰ und R²¹ gleich oder verschieden sind und die oben angegebene Bedeutung von R³ und R⁴ haben,
D für einen Rest der Formel 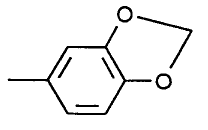 steht,
E für einen Rest der Formel -CH₂-Y-Z- steht,
worin
Y eine Bindung oder ein Sauerstoff- oder Schwefelatom oder die -NH-Gruppe bedeutet,
Z eine Alkylenkette mit bis zu 4 Kohlenstoffatomen bedeutet
und deren Tautomere und Salze.

4. Purin-6-on-derivate nach Anspruch 1 bis 3 zur therapeutischen Anwendung.

5. Verfahren zur Herstellung von Purin-6-on-derivaten nach Anspruch 1 bis 3, dadurch gekennzeichnet, daß man
[A] zunächst Verbindungen der allgemeinen Formel (II) 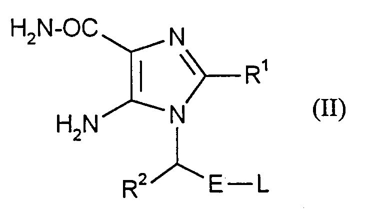 in welcher
R¹, R², E und L die angegebene Bedeutung haben,
durch Umsetzung mit Verbindungen der allgemeinen Formel (III)
D-A-CO-Cl (III)
in welcher
A und D die angegebene Bedeutung haben,
in inerten Lösemitteln und in Anwesenheit einer Base,
in die Verbindungen der allgemeinen Formel (IV) 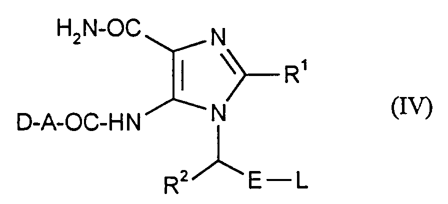 in welcher
A, D, E, L, R¹ und R² die angegebene Bedeutung haben,
überführt,
und in einem zweiten Schritt in inerten Lösemitteln und in Anwesenheit einer Base cyclisiert,
oder
[B] Verbindungen der allgemeinen Formel (II) mit Verbindungen der allgemeinen Formel (V)
D-A-CO₂R²² (V)
in welcher
D und A die oben angegebene Bedeutung haben und
R²² für C₁-C₄-Alkyl steht
in Alkoholen und in Anwesenheit einer Base direkt umsetzt
und gegebenenfalls die unter R² aufgeführten Substituenten durch Acylierung, Oxidation und/oder Azidaustausch einführt bzw. derivatisiert,
und ebenso die oben unter D und L aufgeführten Substituenten nach üblichen Methoden einführt und/oder variiert.

6. Arzneimittel enthaltend Purin-6-on-derivate nach Anspruch 1 bis 3.

7. Arzneimittel nach Anspruch 6 zur Behandlung von cardiovaskulären Erkrankungen, peripheren Gefäßerkrankungen und Erkrankungen des Urogenitalsystems.

8. Verfahren zur Herstellung von Arzneimitteln nach Anspruch 6 und 7, dadurch gekennzeichnet, daß man die Purin-6-on-derivate gegebenenfalls mit Hilfe von üblichen Hilfs- und Trägerstoffen in eine geeignete Applikationsform überführt.

9. Verwendung von Purin-6-on-derivaten nach Anspruch 1 bis 3 zur Herstellung von Arzneimitteln.

10. Verwendung nach Anspruch 9 zur Herstellung von Arzneimitteln zur Behandlung von cardiovaskulären Erkrankungen, peripheren Gefäßerkrankungen und Erkrankungen des Urogenitalsystems.
